# FASCICULE DE BREVET EUROPEEN

(11) **EP 0 680 323 B1**
(45) Date de publication et mention de la délivrance du brevet: **02.04.1997**
(21) Numéro de dépôt: 94904685.8
(22) Date de dépôt: 18.01.1994
(51) Int. Cl.: A61K 31/445, A61K 45/06

(54) **ASSOCIATION SYNERGISANTE AYANT UN EFFET ANTAGONISTE DES RECEPTEURS NK1 ET NK2**
SYNERGISTISHE KOMBINATION MIT EINER ANTAGONISTISCHEN WIRKUNG AUF DEN NK1-UND NK2- REZEPTOREN
SYNERGISING ASSOCIATION HAVING AN ANTAGONIST EFFECT ON NK1 AND NK2 RECEPTORS

(30) Priorité: 19.01.1993 FR 9300451
(43) Date de publication de la demande: 08.11.1995
(73) Titulaire: RHONE-POULENC RORER S.A., 92160 Antony (FR)
(72) Inventeur: GARRET, Claude, F-94120 Fontenay-sous-Bois (FR); MONTIER, François, F-75012 Paris (FR)
(74) Mandataire: Savina, Jacques
(86) Numéro de dépôt international: FR9400055
(87) Numéro de publication internationale: WO9416697

(56) Documents cités:
- EP-A- 0 474 561
- J. PHARMACOL. EXP. THER. vol. 262, no. 2 , 1992 pages 646 - 653 J.L. ELLIS 'Antigen-induced enhancement of non-cholinergic contractile responses to vagus nerve and electrical field stimulation in guinea pig isolated trachea.'
- BR. J. PHARMACOL. vol. 103, no. 2 , 1991 pages 1535 - 1541 C.A. MAGGI 'Tachychinin antagonists and capsaicin-induced contraction of the rat isolated urinary bladder: evidence for tachychinin-mediated cotransmission.'
- TRENDS PHARMACOL. SCI. vol. 13, no. 7 , 1992 pages 266 - 269 K.J. WATLING 'Nonpeptide antagonists herald new era in tachykinin research.'
- J. PHARMACOL. EXP. THER. vol. 264, no. 3 , Mai 1993 pages 1327 - 1332 A. LECCI 'Evidence against a peripheral role of tachychinins in the initiation of micturition reflex in the rats.'
- C.R. Acad. Sci., Paris (1992), tome 314, 199-204, Garret et al

## Description

La présente invention concerne l'association synergisante constituée d'au moins un antagoniste des récepteurs NK1 et d'au moins un antagoniste des récepteurs NK2.

La substance P et la neurokinine A sont des neuropeptides appartenant à la famille des tachykinines. Les tachykinines sont des ligands endogènes connus pour leur capacité à stimuler 3 types de récepteurs appelés NK1, NK2 et NK3.

Les effets de la substance P sont médiés principalement par les récepteurs NK1. Les effets de la neurokinine A sont médiés principalement par les récepteurs NK2.

La substance P est un undécapaptide qui est impliqué dans de nombreuses pathologies telles que par exemple la transmission de la douleur, les désordres du système nerveux central, des phénomènes inflammatoires ou respiratoires ...

La neurokinine A est également impliquée dans de nombreuses pathologies telles que la transmission de la douleur, l'arthrite, l'asthme, les phénomènes inflammatoires, les psychoses, les désordres tensionnels, les désordres vésicaux, les cystites ...

Jusqu'à ces dernières années, malgré les recherches mises en oeuvre et malgré l'intérêt suscité [M.R. Hanley, TINS, (5) 139 (1982)], il n'avait pratiquement pas été découvert de produit agissant spécifiquement sur la substance P ou sur la neurokinine A et ayant une structure non peptidique.

Des antagonistes des récepteurs NK1 (antagonistes des effets de la substance P) sont maintenant connus et décrits notamment dans les demandes de brevet EP 429 366, EP 514 273, EP 514 275, WO 90/05525, WO 90/05729, WO 91/18899, WO 91/09844, WO 92/01688, WO 92/06079, WO 92/15585, WO 92/12151, WO 92/20661, WO 92/20676, WO 92/21677, WO 93/00330, WO 93/00331, WO 93/01159, WO 93/01169, WO 93/01165, WO 93/01170, WO 93/06099, WO 93/09116, WO 93/10073, WO 93/18023, WO 93/19064, WO 93/21155, WO 93/21181, WO 93/23380, EP 499 313, EP 394 989, EP 443 132, EP 482 539, EP 512 902, EP 517 589, EP 520 555, EP 522 808, EP 528 495, EP 532 456, EP 533 280, EP 536 817, EP 545 478, EP 559 538, XIIè Int. Symp. on Med. Chem., Bâle, 13-17 septembre 1992 ou 3rd Meeting of the European Neuropeptide Club (Cambridge 5-7 avril 1993).

Des antagonistes des récepteurs NK2 (antagonistes des effets de la neurokinine A) sont également connus et décrits notamment dans les demandes de brevet EP 428 434, EP 474 561, EP 512 901, EP 515 240, FR 2 678 267, WO 92/19254 ou WO 93/14084.

J.L. Ellis et coll., J. Pharmacol. Exp. Ther., 262(2), 646 (1992) mentionnent l'association d'un antagoniste des récepteurs NK₁ : CP 96345 et d'un peptide antagoniste des récepteurs NK₂ (MEN 10376) mais aucune étude des produits séparés puis associés n'a été faite et aucune synergie d'action découverte.

C.A. Maggi, Br. J. Pharmacol., 103(2), 1535 (1991) décrit l'étude du spantide et du L-659,877 seuls ou associés mais dans la mesure où le spantide est à la fois antagoniste des récepteurs NK₁ et NK₂ cette étude ne peut pas permettre de découvrir l'intérêt d'une association d'un antagoniste des récepteurs NK₁ et d'un antagoniste des récepteurs NK₂.

Il a été également mis en évidence que certains produits sont doués de l'activité antagoniste des récepteurs NK1 et de l'activité antagoniste des récepteurs NK2 : M. Murai et coll., J. Pharm. Exp. Ther., 262(1), 403 (1992). Cependant aucun de ces produits n'a manifesté jusqu'à présent une activité très élevée.

Jusqu'à présent il avait été supposé que les effets des antagonistes des récepteurs NK1 et NK2 pouvaient agir de façon similaire et additive sur certaines fonctions. Il a été maintenant trouvé que les effets des antagonistes des récepteurs NK1 associés à des antagonistes des récepteurs NK2 se potentialisent, ce qui ouvre une voie particulièrement intéressante dans les domaines thérapeutiques où interviennent la substance P et/ou la neurokinine A.

A titre non limitatif, des antagonistes des récepteurs NK1 peuvent être notamment des dérivés de la classe des perhydroisoindoles, des dérivés de la classe des 3-amino-quinuclidines-2-substituées, des dérivés de la classe des amino-azabicycloalcanes, des dérivés de la classe des 3-amino-pipéridines-2-substituées, des dérivés de la classe des 1-azabicyclo[3.2.2]nonan-3-amine, des dérivés de la classe des sels de N-alcoyl-quinuclidinium, les dérivés de la classe des pseudopeptides, les dérivés de la classe des N,N-diacylpipérazines, des dérivés aromatiques substitués, des dérivés dialcoylènepipéridino, des sels quaternaires de pipéridines substituées ...

A titre d'exemple, des produits de la classe des dérivés de perhydroisoindoles peuvent être de structure : 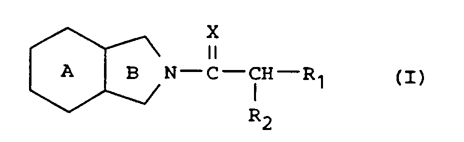 ainsi que leurs sels lorsqu'ils existent, dans laquelle :
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé (5 à 9 atomes de carbone) et un ou plusieurs hétéroatomes choisis parmi l'oxygène, l'azote ou le soufre, et éventuellement substitué par un atome d'halogène ou par un radical alcoyle ou alcoyloxy,
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino ou acylamino,
- le bicycle : 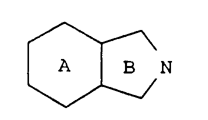 représente un cycle perhydroisoindole ou thiapyranopyrrole de formule générale : 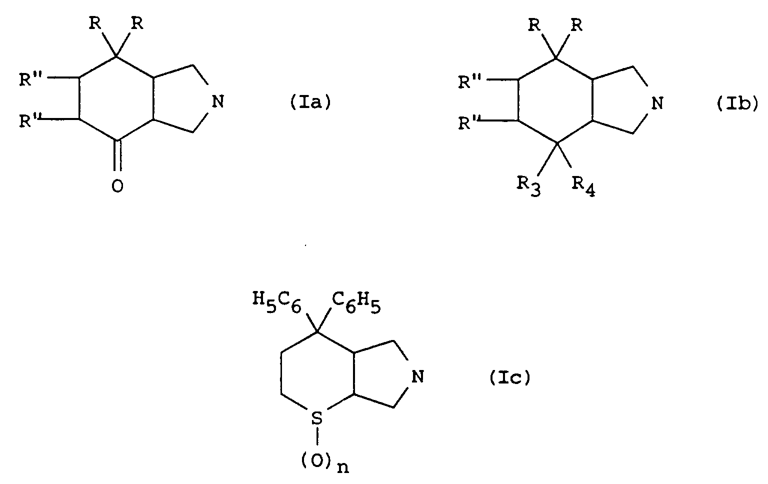 et le symbole X représente un atome d'oxygène ou de soufre ou un radical NR₆ pour lequel R₆ est un atome d'hydrogène, un radical alcoyle contenant 1 à 12 atomes de carbone et éventuellement substitué [par un ou plusieurs radicaux carboxy, dialcoylamino, acylamino, alcoyloxycarbonyle, alcoyloxycarbonylamino, carbamoyle, alcoylcarbamoyle, dialcoylcarbamoyle (les portions alcoyle de ces substituants pouvant être elles même substituées par dialcoylamino ou phényle) ou substitué par des radicaux phényle, phényle substitué (par halogène, alcoyle, alcoyloxy, ou dialcoylamino), naphtyle, thiènyle, furyle, pyridyle ou imidazolyle] ou un radical dialcoylamino, ou bien si le cycle est de formule (Ib) ou (Ic) le symbole X représente un atome d'oxygène, ou un radical NH, ou bien
- le bicycle : 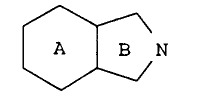 représente un cycle perhydroisoindole ou thiapyranopyrrole de formule générale : 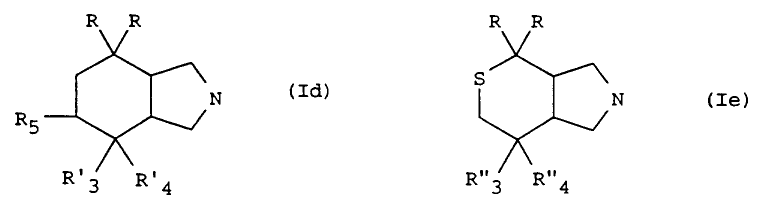 et le symbole X représente un atome d'oxygène.

Dans les formules générales (Ia) à (Ie) :
- les symboles R sont identiques et représentent des radicaux phényle éventuellement substitués par un atome d'halogène ou par un radical méthyle en position 2 ou 3,
- les symboles R'' sont identiques et représentent des atomes d'hydrogène ou forment ensemble une liaison,
- le symbole R₃ représente un atome d'halogène ou un radical hydroxy et le symbole R₄ représente un atome d'hydrogène ou simultanément à R₃ représente un atome d'halogène.
- le symbole R'₃ représente un radical phényle éventuellement substitué en position -2 par un radical alcoyle ou alcoyloxy (1 ou 2 atomes de carbone), et le symbole R'₄ représente un atome de fluor ou un radical hydroxy, et le symbole R₅ représente un atome d'hydrogène ou bien les symboles R'₄ et R₅ représentent des radicaux hydroxy, ou bien le symbole R'₄ forme avec R₅ une liaison.
- le symbole R''₃ représente un atome d'hydrogène et le symbole R''₄ représente un atome de fluor ou un radical hydroxy, ou bien le symbole R''₃ représente un radical phényle éventuellement substitué en position -2 par un radical alcoyle ou alcoyloxy (1 ou 2 atomes de carbone), et le symbole R''₄ est un radical hydroxy, ou bien les symboles R''₃ et R''₄ forment ensemble un radical oxo.
- le symbole n est un nombre entier de 0 à 2 ;
   étant entendu que les produits de formule générale (Ia) à (Id) peuvent présenter des formes stéréoisomères qui sont décrites plus en détail dans les demandes de brevet européen EP 429 366, EP 514 273 et EP 514 275 ou dans la demande internationale WO 93/21155, et que les produits de formule générale (Ie) se présentent sous la forme (3aS,7R,7aR), (3aR,7S,7aS) ou (3aRS,7SR,7aSR), si R₄ est hydroxy et R₃ est un radical phényle, ou se présentent sous la forme (3aR,7S,7aS), (3aS,7R,7aR) ou (3aRS,7SR,7aSR) ou leurs mélanges, si R₃ est hydrogène et R₄ est hydroxy, ou se présentent sous la forme (3aR,7R,7aS), (3aS,7S,7aR) ou (3aRS,7RS,7aSR) ou leurs mélanges, si R₃ est hydrogène et R₄ est fluor, ou se présentent sous la forme (3aS,7aR), (3aR,7aS) ou (3aRS,7aSR) ou leurs mélanges, si R₃ forme avec R₄ un radical oxo.

A titre d'exemple des produits de la classe des 3-amino-cuinuclidines-2-substituées, des dérivés de la classe des amino-azabicycloalcanes, des dérivés de la classe des 3-amino-pipéridines-2-substituées, des dérivés de la classe des 1-azabicyclo[3.2.2]nonan-3-amine, des dérivés de la classe des sels de N-alcoyl-quinuclidinium peuvent être des produits de formule générale : 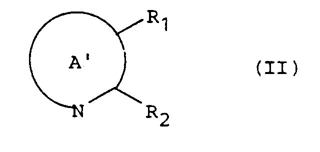 sous leurs formes stéréoisomères ou leurs mélanges, dans laquelle le cycle : 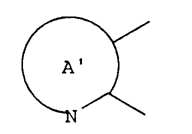 représente un hétérocycle azoté monocyclique ou un polycyclique condensé de structure :

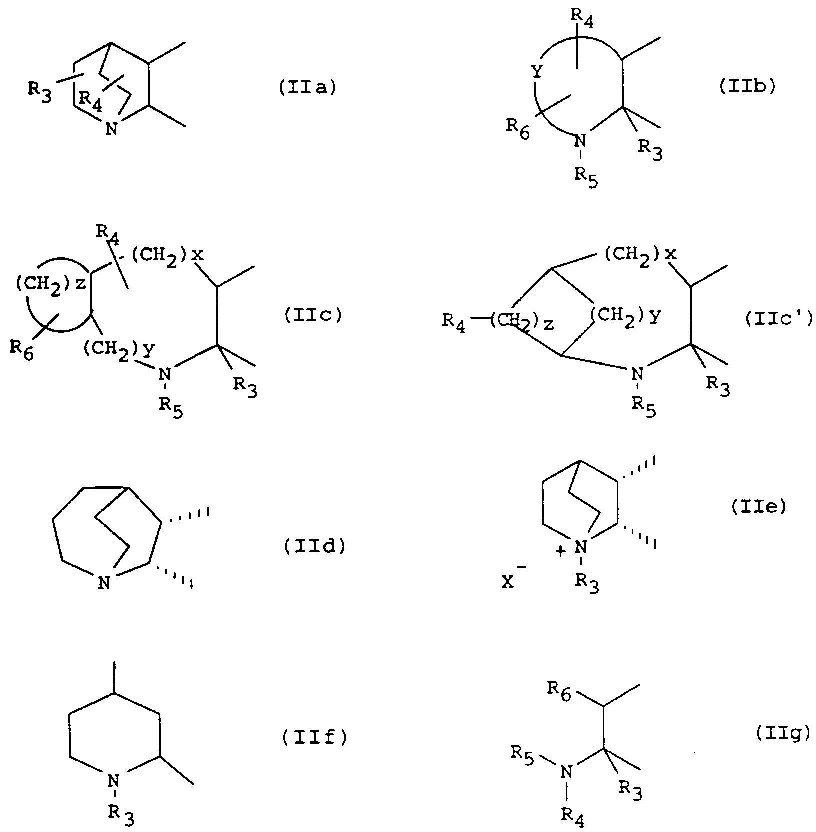

Lorsque le cycle A' répond à la formule générale (IIa) :
1) R₄ est un atome d'hydrogène,
   a₁) R₁ est un radical =N-CH₂-R ou -N=CH-R, pour lequel
      R est cycloalcoyle (5 à 7 atomes de carbone), norbornyle, pyrrolyle, thiényle, pyridyle, indolyle, biphényle ou phényle pouvant être substitués par 1 ou 2 substituants choisis parmi fluor, chlore, brome, trifluorométhyle, alcoyle ou alcoyloxy (1 à 3 atomes de carbone), carboxy, alcoyloxycarbonyle dont la partie alcoyle contient 1 à 3 atomes de carbone ou benzyloxycarbonyle, et
      R₂ est un radical Ar-CHR'- pour lequel
      R' est un radical alcoyle ramifié (3 ou 4 atomes de carbone), un radical alcoylène ramifié (5 ou 6 atomes de carbone), un radical cycloalcoyle (3 à 7 atomes de carbone), furyle, thiényle, pyridyle, indolyle, biphényle ou phényle pouvant être substitués par 1 ou 2 substituants choisis parmi fluor, chlore, brome, trifluorométhyle, alcoyle ou alcoyloxy (1 à 3 atomes de carbone), carboxy, alcoyloxycarbonyle dont la partie alcoyle contient 1 à 3 atomes de carbone ou benzyloxycarbonyle, et Ar est un radical phényle ou bien
   a_{2.1}) R₁ est un radical -NH-CH₂-R pour lequel
      R est défini comme ci-dessus et R₂ est défini comme précédemment, ou
   a_{2.2}) R représente un radical phényle disubstitué par un radical méthoxy et un radical butyle ramifié, si R₂ est un radical benzhydryle, ou bien
   a_{2.3}) R représente un radical phényle trisubstitué par des radicaux X₁ à X₃ pour lesquels X₁ est H, alcoyle ou alcoyloxy (1 à 10 atomes de carbone) éventuellement substitués par 1 à 3 atomes de fluor et, X₂ et X₃ sont H, halogène NO₂, alcoyle ou alcoyloxy (1 à 10 atomes de carbone) éventuellement substitués par 1 à 3 atomes de fluor, OH, CN, phényle, NH₂, alcoylamino, dialcoylamino, alcoylcarbamoyle, alcoylcarbamoylalcoyle ou acylamino (dont les parties alcoyle ou acyle contiennent 1 à 6 carbones), ou alcoyloxyalcoyle (dont les parties alcoyle contiennent 1 à 4 atomes de carbone) si R₂ est soit Ar-CHR'- pour lequel R' est défini comme ci-dessus ou représente alcoyle ou alcoyloxy (1 à 10 atomes de carbone) éventuellement substitués par 1 à 3 atomes de fluor, et Ar est un radical phényle, soit un radical phényle, biphényle, naphtyle, pyridyle, thiényle ou furyle pouvant être mono à trisubstitués par halogène ou alcoyle ou alcoyloxy (1 à 10 atomes de carbone) éventuellement substitués par 1 à 3 atomes de fluor, et
      R₃ est un atome d'hydrogène, ou bien
   a₃) R₁ est -NH-(C=Y)-R pour lequel R est un dérivé du benzopyranne contenant éventuellement un autre hétéroatome (O, S ou NR' pour lequel R' est H, alcoyle ou aralcoyle) ou un dérivé du benzofuranne éventuellement substitués en α de l'oxygène par des radicaux méthyle, et/ou substitués par un radical oxo ou thioxo et/ou substitués sur le noyau phényle par halogène, alcoyle, haloalcoyle, aralcoyle, alcoyloxy, aralcoyloxy, acyle, acyloxy, OH, NH₂, CN, NO₂, -NH-CO-R'', s(O)n-R'', -NH-SO₂R'', COOR'', CONR''R''', OCONR''R''', CSNR''R''', SO₂NR''R''', pour lesquels R'' et R''' sont H, alcoyle, phényle ou aralcoyle, et Y est O, S ou 2H,
      R₂ est -CHR₅R₆ pour lequel R₅ est thiényle ou phényle et R₆ est alcoyle, alcényle, cycloalcoyle, furyle, thiényle, pyridyle, indolyle, biphényle ou phényle, et R₃ est H ou alcoyle;
   b₁) R₁ est défini comme en a₁) ou en a_{2.1}) et R est défini comme en a₁) ou représente 2,3-dihydrobenzofuranyle, alcoyloxythiényle dont la partie alcoyle contient 1 à 3 atomes de carbone, hydroxypyridyle, quinolyle, naphtyle, alcoyloxynaphtyle dont la partie alcoyle contient 1 à 3 atomes de carbone, 2,3-méthylènedioxyphényle, phényle substitué par 1 ou 2 radicaux choisis parmi les radicaux cités précédemment ou cyano, nitro, amino, N-monoalkylamino (1 à 3 atomes de carbone), allyle, hydroxy, carboxybenzyloxy, alcoyloxycarbonylbenzyloxy dont la partie alcoyle contient 1 à 3 atomes de carbone, carboxamido ou N,N-dialcoylcarboxamido dont les parties alcoyle contiennent 1 à 3 atomes de carbone, et
      R' est défini comme ci-dessus ou représente phényle substitué par 1 ou 2 radicaux choisis parmi les radicaux cités précedemment ou phénylalcoyle dont la partie alcoyle contient 1 à 3 atomes de carbone, allyloxy ou hydroxy, et
      R₃ est un atome d'hydrogène ou un radical alcoyle (1 à 4 atomes de carbone) situé en position 5, ou bien
   b₂) R₁ est défini comme en a_{2.3}) et R₂ est phényle, biphényle, naphtyle, pyridyle, thiényle ou furyle pouvant être mono à trisubstitués par halogène ou alcoyle ou alcoyloxy (1 à 10 atomes de carbone) éventuellement substitués par 1 à 3 atomes de fluor, et
      R₃ est un radical alcoyle (1 à 6 atomes de carbone) , ou bien
   c) R₁ est un radical -NH-CH₂-R pour lequel
      R est phényle, thiényle, pyridyle ou furyle éventuellement substitués par 1 à 3 substituants choisis parmi fluor, chlore, brome, iode, trifluorométhyle, alcoyle ou alcoyloxy (1 à 4 atomes de carbone),
      R₂ est un radical phényle, naphtyle, thiényle, pyridyle ou furyle éventuellement substitués comme défini ci-dessus pour R, et
      R₃ est un atome d'hydrogène ou un radical alcoyle (1 à 6 atomes de carbone) situé en position 5 ou 6, ou bien
   d) R₁ est un radical -NH-CH₂-R pour lequel R est phényle, naphtyle, pyridyl, quinolyle, thiényle, furyle, phénoxyphényle, oxazolyle, tétrazolyle, thiazolyle, imidazolyle ou pyrazolyle éventuellement substitués par des substituants alcoyle ou alcoyloxy portant éventuellement 1 à 3 halogènes, alcoylthio, alcoylsulfinyle, alcoylsulfonyle, alcoylsulfonylamino, dialcoylamino dont les parties alcoyle peuvent être substituées par alcoylsulfinyle ou alcoylsulfonyle (dont les parties alcoyle contiennent 1 à 6 carbones), sulfonamido ou alcènyle (2 à 6 carbones), ou pour lequel R est phényle substitué par halogène, alcynyle, alcoylamino, N-alcoyl N-alkylsulfonyl amino ou N-alcoyle N-alcanoyl amino (pouvant être substitués par halogène sur la partie alcoylsulfonyle ou alcanoyle), alcoylsulfonylamino ou alcanoylamino dont les parties alcoyle peuvent être substituées par halogène,
      R₂ est un radical Ar-CH(Ar')- pour lequel Ar et Ar' sont des radicaux cycliques tels que R ci-dessus, pouvant être substitués par alcoyle, alcoyloxy, alcoylthio, alcoylsulfinyle, alcoylamino (dont les parties alcoyle contiennent 1 à 4 carbones), trifluorométhyle ou trifluorométhoxy et
      R₃ est alcoyle éventuellement substitué (par alcoyloxy, carbamoyle, -CONRₐR_{b}, -COOH, -COORₐ, -CHRₐOR_{b}, CHRₐNR_{b}R_{c}, -CORₐ, -CONRₐOR_{b}, ou aryle tel que défini pour R éventuellement substitué, les radicaux alcoyle ayant 1 à 6 carbones et Ra, Rb et Rc étant H, alcoyle, alcoyloxy, cycloalcoyle ou aryle éventuellement substitués), alcènyle (2 à 6 carbones), cycloalcoyle (3 à 8 carbones), ou un radical tel que défini ci-dessus pour les substituants du radical alcoyle, ou un radical Y-(CH₂)ₘ-CHR_{d}-(CH₂)ₙ-NR'_{d}-CO- pour lequel Y est -CN, -CH₂Z ou -COZ (pour lequel Z est OH, NH₂, -Oalcoyle, -NHalcoyle ou -N(alcoyle)₂), R_{d} est H, alcoyle, benzyle, ou -(CH₂)ₚ-Y et R'_{d} est défini comme R_{d} ou alcoyle substitué (par OH, NH₂-, -SCH₃, SH) ou 4-hydroxybenzyle ou 3-indolylméthyle.
2) R₃ en position 5 forme avec R₄ une chaine alcoylène contenant 3 à 5 atomes de carbone si R₄ est en position 6, ou contenant 2 ou 3 atomes de carbone si R₄ est en position 7, ou contenant 2 à 4 atomes de carbone si R₄ est en position 8, ou bien lorsque R₃ est en position 5 et R₄ en position 6, R₃ et R₄ peuvent former une chaîne : 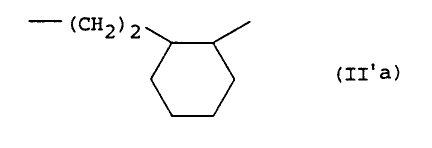 ou bien lorsque R₄ est en position 8 et R₃ en position 5, R₃ et R₄ peuvent former une chaîne : -CH2-X-CH2- dans laquelle X est O, S, NH ou >N(alcoylamino) dont la partie alcoyle contient 1 à 3 atomes de carbone,
   R₁ a la structure définie en 1a_{2.1}) et R est défini comme en 1a_{2.3}) ou représente cycloalcoyle (5 à 7 atomes de carbone), pyrrolyle, thiényle, pyridyle, phényle pouvant être substitués par 1 à 3 substituants choisis parmi fluor, chlore, brome, trifluorométhyle, alcoyle ou alcoyloxy (1 à 3 atomes de carbone), carboxy, alcoyloxycarbonyle dont la partie alcoyle contient 1 à 3 atomes de carbone ou benzyloxycarbonyle, et
   R₂ est un radical -CHR'R'' pour lequel R' est un radical furyle, thiényle, pyridyle, indolyle, biphényle ou phényle pouvant être substitué par 1 ou 2 substituants choisis parmi halogène, alcoyle ou alcoyloxy (1 à 10 atomes de carbone) pouvant être substitués par 1 à 3 fluor, carboxy, alcoyloxycarbonyle (la partie alcoyle contenant 1 à 3 carbones) ou benzyloxycarbonyle, et R'' est thiényle, phényle, halogénophényle, phényle substitué par alcoyle ou alcoyloxy (1 à 10 atomes de carbone) pouvant être substitués par 1 à 3 fluor.

Lorsque le cycle A' répond à la formule générale (IIb) :
1) Y est (CH₂)ₙ pour lequel n égale 1 à 6 et peut représenter une chaîne insaturée,
   R₁ est un radical -NR'-CH₂-R pour lequel R est aryle choisi parmi phényle, naphtyle, indanyle, thiényle, pyridyle, furyle, thiazolyle, isothiazolyle, oxazolyle, isoxazolyle, triazolyle, tétrazolyle et quinolyle éventuellement substitué par 1 ou plusieurs substituants choisis parmi halo, nitro, alcoyle, alcoyloxy, trifluorométhyle, amino, phényle, alcoylamino, formamido, acylamido, acylamidoalcoyle, alcoylcarbamoyle ou cycloalcoyle (3 à 7 atomes de carbone) éventuellement substitué par 1 ou 2 substituants tels que cités ci-dessus et dans lequel l'un des atomes de carbone peut être remplacé par N, O ou S, et R' est un atome d'hydrogène ou un radical alcoyle, ou R₁ est défini comme en 1a_{2.3})
   R₂ est un atome d'hydrogène, un radical alcoyle droit ou ramifié, phénylalcoyle, benzhydryle ou un radical aryle ou cycloalcoyle tel que définis précédemment pour R ou aryle tel que thiazolyle, isothiazolyle, oxazolyle, isoxazolyle triazolyle ou tétrazolyle, ces différents cycles pouvant être substitués par 1 ou plusieurs substituants choisis parmi les substituants cités précédemment pour R, ou choisis parmi dialcoylamino, alcoyloxycarbonyle, alcoyloxycarbonylalcoyle, acyloxy, acylalcoyloxy, acyle ou acylalcoyle,
   R₃ est un atome d'hydrogène ou lorsque R₁ est défini comme en 1a_{2.3}) peut être un radical phényle ou alcoyle (1 à 6 carbones), ou bien R₂ et R₃ forment ensemble avec le carbone auquel ils sont attachés un carbocycle saturé de 3 à 7 chaînons dans lequel les atomes de carbone peuvent être éventuellement remplacés par O, N ou S,
   R₅ est un radical -(CHR'')ₘ-R''' pour lequel m est un entier de 0 à 6, chacun des motifs CHR'' pouvant former avec le motif adjacent une double liaison, et tous les R'' n'étant pas nécessairement identiques,
   R₄ et R₆ peuvent être des atomes d'hydrogène ou des radicaux hydroxy, halo, amino, alcoylamino, dialcoylamino, alcoyloxy, alcoyloxycarbonyle, alcoyloxycarbonylalcoyle, acyloxy, acylalcoyloxy, acyle ou acylalcoyle ou des radicaux tels que cités précédemment pour la définition de R₂, sous réserve de ne pas pouvoir former de cycle avec R₅, ou si R₁ est défini comme en 1a_{2.3)} R₄ et R₆ peuvent aussi être oxo, hydroxyalcoyle ou alcoyloxyalcoyle, et
   R'' et R''' sont définis comme R₄ et R₆, ou si R₁ est un radical défini comme en 1a2.3), R'' peut aussi être =N-OH et R''' un radical -NHCOR_{d}, -NHCH₂R_{d}, SO₂R_{d} ou NHSO₂R_{d}, et R_{d} est H, alcoyle, phénylalcoyle ou phényl substitué par alcoyle (les parties alcoyle ayant 1 à 6 carbones), ou bien
2) -Y-N(R₅)- est le résidu d'un système azabicyclique (par exemple quinuclidinyle, azabicycloheptanyle, azabicyclooctanyle),
   a) R₁ est un radical -X-CH₂-R pour lequel X est un atome d'oxygène ou de soufre, et R est un radical phényle éventuellement substitué par 1 à 3 radicaux identiques ou différents choisis parmi alcoyle, alcényle ou alcynyle (2 à 6 atomes de carbone), halogène, cyano, nitro, trifluorométhyle, triméthylsilyle, -OR', -SCH₃, -SOCH₃, -SO₂CH₃, -NR'R'', -NR'COR'', -NR'CO₂R'', -CO₂R' ou -CONR'R'' pour lesquels R' et R'' identiques ou différents représentent hydrogène, alcoyle, phényle ou trifluorométhyle,
      R₂ représente un radical -CRₐR_{b}R_{c} pour lequel Rₐ et R_{b} identiques ou différents représentent phényle ou thiényle éventuellement substitués par halo ou trifluorométhyle, ou cycloalcoyle, ou Ra peut aussi représenter phényle ou thiényle substitués par alcoyloxy ou Rb peut aussi représenter benzyle pouvant être substitué comme défini précédemment et R_{c} est hydrogène ou hydroxy, et
      R₃, R₄ et R₆ sont H, ou bien
   b) R₁ est un radical -CH₂-CHX-Ar ou =CH-CHX-Ar pour lequel Ar est un radical phényle pouvant être substitué comme défini ci-dessus en a), X est H, OH, =O ou halogène,
      R₂ est un radical -CRₐR_{b} pour lequel Rₐ et R_{b} identiques ou différents représentent phényle, thiényle ou benzyle éventuellement substitués par halo ou trifluorométhyle, et
      R₃, R₄ et R₆ sont H, ou bien
   c) R₁ est un radical -NR''-CH₂-R pour lequel R est défini comme en IIb 1) [pour R' est H ou alk] ou représente alcoyloxycarbonyle, formyle, hydroxyméthyle, phénoxyméthyle ou alcoyloxyméthyle,
      R₂ est défini comme en IIb 1),
      R₃, R₄, R₆ et R'' sont H, alcoyle ou phényle, ou bien
   d) -Y- substitué par des radicaux R₄ et R₆ est une chaîne -CHRa-CRb(CRcRd) - dans laquelle l'un de Ra est H, hydroxyméthyle, alcoyle, acyloxyalcoyle, alcoyloxyméthyle ou benzyloxyméthyle, Rb et Rc sont H, alcoyle ou phényle, et Rd est méthyle ou hydroxyméthyle,
      R₁, R₂ et R₃ sont définis comme ci-dessus en c) et
      R₅ est H, benzyle ou un radical [(CH₂)ₘRe]-Rf pour lequel Re et Rf sont définis comme R' et R'' ci-après en IIg);
3) R₁ est défini comme pour la formule (IIb)-2a) étant entendu que R dans -X-CH₂-R peut aussi porter un substituant SR', SOR' ou SO₂R',
   R₂ est un radical phényle, naphtyle, indazolyle, thiényle, furyle, pyridyle, thiazolyle, tétrazolyle ou quinolyle pouvant être substitués par alcoyle, alcoyloxy, halogène ou trifluorométhyle, ou un radical benzhydryle ou benzyle, ces radicaux pouvant être substitués par alcoyle ou alcoyloxy (1 à 6 carbones), halogène ou CF₃,
   Y est -(CH₂)ₙ- pour lequel n égale 1 à 3,
   R₃ est H,
   R₄ et R₆ sont H, halogène, -CH₂OR°, alcoyle, oxo, COOR° ou CONR°R° pour lesquels R° est H, CF₃, alcoyle ou phényle, et
   R₅ est H, alcoyle éventuellement substitué [par COOR°°, CONR°°R°°, OH, CN, COR°, -NR°°R°°, C(NOH)NR°°R°°, CONHphénylalcoyle, COCOOR°°, COCONR°°R°°, phényle éventuellement substitué (par 1 ou plusieurs substituants alcoyle, alcoyloxy, halogène ou CF₃), ou par un hétérocycle aromatique lui-même pouvant être substitué], R° est défini comme précédemment et R°° est H ou alcoyle;
   étant entendu que sauf mention spéciale, les radicaux et parties alcoyle cités ci-dessus contiennent de 1 à 6 atomes de carbone),

Lorsque le cycle A' répond à la formule générale (IIc) :
x et y sont des entiers de 0 à 4 et z est un entier de 0 à 6, étant entendu que le cycle contenant (CH₂)_{z} peut contenir jusqu'à 3 doubles liaisons et l'un des (CH₂)_{z} peut être remplacé par O, S ou N,
R₁ est défini comme dans le cas où A' est de formule (IIb)-1), R est défini comme dans le cas où A' est de formule (IIb)-1) ou représente thiazolyle, isothiazolyle, oxazolyle, isoxazolyle, triazolyle ou tétrazolyle, éventuellement substitués par 1 ou plusieurs substituants, les substituants étant choisis parmi les radicaux cités précédemment pour la formule générale (IIb)-1) ou phényle, alcoylcarbamoyle ou alcoylcarbamoylalcoyle et R' est défini comme dans le cas où A' est de formule (IIb)-1) ou représente un radical alcoyle substitué par hydroxy, alcoyloxy ou fluoro,
R₂ et R₃ sont définis comme pour (IIb)-1),
R₅ est un radical - (CHR'')ₘ-R''' pour lequel m est un entier de 0 à 12, chacun des motifs CHR'' pouvant former avec le motif adjacent une double ou une triple liaison, et tous les R'' n'étant pas nécessairement identiques, ou bien R₅ est défini comme précédemment pour la formule (IIb)-1) et
R₄, R₆, R'' et R''' sont définis comme pour la formule (IIb)-1) ou peuvent être des radicaux carboxy ou carboxyalcoyle,
étant entendu que sauf mention spéciale, les radicaux et parties alcoyle cités ci-dessus contiennent de 1 à 6 atomes de carbone.

Lorsque le cycle A' répond à la formule générale (IIc') :
R₁ est défini comme précédemment en (IIc), le radical R de -NR'-CH₂-R pouvant également porter un substituant dialcoylamino,
R₂ est défini comme précédemment en (IIc), étant entendu que les radicaux phényle du benzhydryle peuvent éventuellement être remplacées par naphtyle, thiényle, furyle ou pyridyle,
R₃ est défini comme précédemment en (IIc), ou représente phényle,
R₄ est défini comme précédemment en (IIc) à l'exception de porter un substituant COOH ou carboxyalcoyle, ou peut être substitué par un radical oxo ou nitrile, ou des groupes tels que définis pour R₂,
R₅ est défini comme précédemment en (IIc) pour lequel R''' est hydroxyimino, ou l'un des groupes cités précédemment pour R₂ et R₄, et R'' est -NHCO-R°, -NHCH₂R°, -NHSO₂R° ou l'un des groupes cités précédemment pour R₂ et R₄, et R° est H, alcoyle, phényle, ou phénylalcoyle, et m est 0 à 8,
x est 0 à 2, y et z sont 1 à 4 et n'importe lequel des atomes de carbone de (CH₂)_{z} peut être substitué par le radical R₄.

Lorsque le cycle A' répond à la formule générale (IId) :
R₁ est un radical -NH-CH₂-R pour lequel R est un radical phényle éventuellement substitué par 1 ou 2 radicaux identiques ou différents choisis parmi les atomes de fluor, de chlore, de brome, ou les radicaux trifluorométhyle, alcoyle, alcoyloxy, carboxy, alcoyloxycarbonyle ou benzoyloxy les radicaux et parties alcoyle (1 à 3 atomes de carbone) ;
R₂ est un radical benzhydryle dont les noyaux phényle peuvent être substitués par 1 ou 2 radicaux tels que cités ci-dessus.

Lorsque le cycle A' répond à la formule générale (IIe) :
R₁ est défini comme ci-dessus lorsque le cycle A' répond à la formule générale (IId) et R est phényle, thiényle, furyle ou pyridyle éventuellement substitué par 1 à 3 substituants choisis parmi cyano, nitro, amino, N-alcoylamino, fluor, chlore, brome, trifluorométhyle, alcoyle, alcoyloxy, allyloxy, alcoyloxycarbonyle, carboxamido, N,N-dialcoylcarboxamido, les radicaux et parties alcoyle (1 à 3 atomes de carbone) ;
R₂ est un radical benzhydryle et
R₃ est un radical alcoyle (1 à 4 atomes de carbone), allyle, phénylalcoyle dont la partie alcoyle contient 1 à 6 atomes de carbone, carboxyalcoyle dont la partie alcoyle contient 1 à 10 atomes de carbone ou alcoyloxycarbonylalcoyle dont la partie alcoyloxy contient 1 à 4 atomes de carbone et la partie alcoyle contient 1 à 10 atomes de carbone et
X est un contre-ion pharmaceutiquement acceptable, choisi parmi chlorure, fluorure, bromure, iodure, mésylate, tosylate ou trifluorométhanesulfonate.

Lorsque le cycle A' répond à la formule générale (IIf) :
R₁ est un radical -X-NR'-X'-R pour lequel X est alcoyle, CO, ou une liaison, X' est CO, oxoalcoyle, oxo(aza)alcoyle ou alcoyle éventuellement substitué par phényle, hydroxyméthyle, COOH pouvant être estérifié ou sous forme d'amide, ou substitué en α par OH, R' est H, alcoyle, carbamoyle, ou alcanoyle ou alcénoyle éventuellement substitués par COOH pouvant être estérifié ou sous forme d'amide, et R est aryle éventuellement substitué ou hétérocyclyle éventuellement partiellement hydrogéné,
R₂ est un radical -X''-R'' pour lequel X'' est méthylène, éthylène, une liaison, carbonyle éventuellement sous forme de cétal ou hydroxyméthylène éventuellement éthérifié et R'' est cycloalcoyle, ou aryle ou hétérocyclyle éventuellement substitués, et
R₃ est aralcoyle, aralcoyloxyalcoyle, hétéroaralcoyle, aroyle, hétéroaroyle, cycloalcoylcarbonyle, aralcanoyle, hétéroaralcanoyle, aralcoyloxycarbonyle ou arylcarbamoyle pouvant être substitués, ou le reste acyle d'un aminoacide éventuellement N-substitué par alcoyle ou carbamoylalcanoyle, ainsi que leurs formes stéréoisomères;

Lorsque le cycle A' est remplacé par la formule générale (IIg) :
R₁ est -NH-CH₂-R pour lequel R est défini comme précédemment en IIb-1) (lorsque R' est H), ou représente alcoyloxycarbonyle, formyle, hydroxyméthyle, phénoxyméthyle ou alcoyloxyméthyle,
R₂ est H, alcoyle, phényle éventuellement substitué par 1 ou plusieurs substituants choisis parmi halogène ou alcoyle ou alcoyloxy éventuellement substitués (par 1 à 3 fluors),
R₃ est défini comme ci-dessus R [en IIb-1)] à l'exception de représenter aminoalcoyle ou alcoylcarbamoyle, ou représente alcoyle ou phénylalcoyle ou benzhydryle pouvant être substitués sur le phényle par un substituant tel que dans la définition ci-dessus,
R₄ est -[(CH₂)ₘR']-R'' pour lequel R' et R'' sont définis comme R₃ ou représentent OH, halogène, COOH, carboxyalcoyle, alcoylamino, dialcoylamino, alcoyloxy, alcoyloxycarbonyle, alcoyloxycarbonylalcoyloxy, acyloxy, acylalcoyloxy ou acyle et m est 0 à 12,
R₅ est H, alcoyle, cycloalcoyle (6 à 10 carbones) contenant 2 cycles condensés ou pontés, benzyle dont la partie phényle peut être substituée par 1 ou plusieurs substituants choisis parmi halogène ou alcoyle ou alcoyloxy éventuellement substitués (par 1 à 3 fluors),
R₆ est H, cycloalcoyle (3 à 8 carbones), alcoyle ou phényle éventuellement substitué par 1 ou plusieurs substituants choisis parmi halogène ou alcoyle ou alcoyloxy éventuellement substitués (par 1 à 3 fluors),
étant entendu que, sauf mention spéciale, les portions alcoyle contiennent 1 à 6 carbones;
et parmi ces produits plus spécialement le 1-N-cyclohexyl-1-phényle-2-N' [(2-méthoxyphényl)méthyl]-1,2-éthanediamine.

A titre d'exemple, des produits de la classe des pseudopeptides peuvent être notamment :
A) des produits de formule générale : 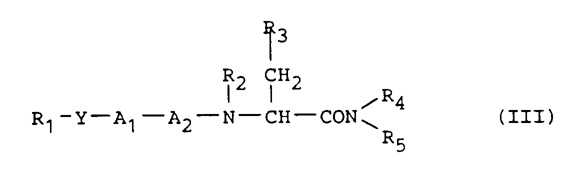 dans laquelle :
   1) A₁ est un radical carbonyle,
      A₂ est le résidu d'un aminoacide, à l'exception de D-Trp, qui peut être substitué,
      R₁ est un radical alcoyle, aryle, arylamino, pyridyle, pyrrolyle, pyrazolopyridyle, quinolyle ou un radical de formule générale : 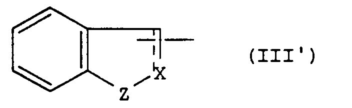 dans laquelle Z est O, S ou NH et X est -CH- ou N, pouvant être éventuellement substitués (notamment par alcoyle) et --- est une liaison simple ou double,
      Y est une liaison ou un radical alcoylène ou alcénylène,
      R₂ est H ou alcoyle,
      R₃ est un radical phényle éventuellement substitué par hydroxy en position 4 ou par 1 ou 2 substituants choisis parmi alcoyle, pouvant porter jusqu'à 3 halogènes, amino, acylamino, carboxyalcoyloxy éventuellement estérifié, halogène, alcoyloxy ou nitro, et
      R₄ est alcoyle éventuellement substitué, et
      R₅ est aralcoyle éventuellement substitué ou pyridylalcoyle, ou bien R₄ et R₅ sont liés ensemble pour former une chaîne alcoylène benzo condensée,
      étant entendu que la nature des substituants et des résidus d'amino acides et la longueur des chaînes carbonées sont tels que définis dans la demande de brevet européen EP 394 989 ou EP 482 539, ou bien
   2) A₁ est un radical carbonyle ou sulfonyle,
      A₂ est un reste 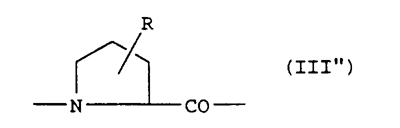 pour lequel R est un radical hydroxy ou alcoyloxy,
      Y est une liaison ou un radical alcènylène,
      R₁ est un radical aryle, ou un radical de formule (III') pour lequel Z est O ou NR₆, R₆ étant H ou alcoyle, et --- est une double liaison,
      R₂ est un atome d'hydrogène,
      R₃ est un radical naphtyle,
      R₄ est un atome d'hydrogène ou un radical alcoyle éventuellement substitué, et
      R₅ est aralcoyle éventuellement substitué,
      étant entendu que la nature des substituants et la longueur des chaînes carbonées sont telles que définies dans la demande de brevet européen EP 443 132.
B) des produits de formule générale : 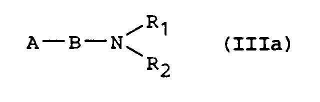 dans laquelle :
   R₁ et R₂ sont H, alcoyle (1 à 6 carbones), cycloalcoyle (3 à 7 carbones) ou benzyle,
   B est le reste d'un amino-acide aromatique, et
   A est soit un radical de structure : 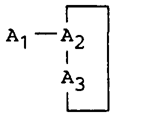 dans laquelle A₁ est une liaison, un résidu 2-azabicyclo[2.2.2]octane-3-carbonyle, leucine, β-naphtylalanine, tryptophane éventuellement protégé, A₂ est un résidu aspartique ou glutamique et A₃ est un résidu (1,2,3,4)-tétrahydroisoquinoléine-3-carbonyle, 2-azabicyclo [2.2.2]octane-3-carbonyle, méthylphénylalanine, arginine, arginine protégée, 6,7-dihydroxy-(1,2,3,4)-tétrahydroisoquinoléine, spinacine, 4-hydroxyproline, β-naphtylalanine ou proline, étant entendu que la liaison -CO-NH- entre A₁ et A₂ ou entre A₂ et B peut être remplacée par -CH₂-NH- ou -CH₂-S- lorsque A₁ est une liaison,
   soit A est un résidu peptidique de structure :

   -A₄-A₅-A₆-P

   pour lequel A₄ et A₅ sont 2-azabicyclo [2.2.2]octane-3-carbonyle ou β-naphtylalanine ou A₅ est une liaison ou un résidu phénylalanine, A₆ est une liaison, tryptophane éventuellement protégé par formyle ou méthyle ou 2-azabicyclo [2.2.2]octane-3-carbonyle, étant entendu que lorsque A₅ et A₆ ne sont pas des liaisons, la liaison CO-NH qui les sépare peut être remplacée par -CH₂-NH- ou -CH₂-S-, et
   P est H ou un groupement protecteur d'amine tel que benzyloxycarbonyle, t.butoxycarbonyle, 3-indolylecarbonyle, benzhydrylcarbonyle ou fluorénylméthyloxycarbonylecarbonyle, ainsi que leurs formes stéréoisomères.

A titre d'exemple, des produits de la classe des diacylpipérazines peuvent être notamment des produits de formule générale : 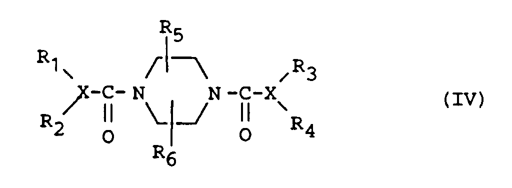 dans laquelle
X est -CH< ou -N<,
R₁ est H, alcoyle (1 à 8 carbones), phényle ou phénylalcoyle substitués (sur le cycle) par alcoyle, halogène, OH, CF₃, NH₂, NHalcoyle, N(alcoyle)₂, -COOH ou COOalcoyle,
R₂ est défini comme R₁ ou -CH₂-R₁ ou représente cycloalcoyle (3 à 7 carbones),
R₃ et R₄ indépendemment sont phényle tel que défini pour R₁, alcoyle (1 à 6 carbones) pouvant être substitué comme phényle ci-dessus ou substitué par SOₓ-alcoyle, pour lequel x est 0 à 2 ou cycloalcoyle (3 à 7 carbones), ou bien les phényles formés par R₃ et R₄ peuvent être fusionnés en ortho pour former un groupe tricyclique avec X, ou bien XR₁R₂ peut être -OR₂,
R₅ et R₆ sont alcoyle, COOR', -CH₂OCOR', -CH₂OH, -CH₂OR'', -CH₂S(O)ₓR'', -CH₂OCONR'R'', -CH₂CONR'R'', -CONR'R'', -COOR°, -CH₂COOR', -CH₂COOR°, -CONHSO₂R°°, -CH₂NR'CONR'R'', ou méthyle substitué par amino, alcoylamino ou dialcoylamino et R' est H ou alcoyle, R'' est défini comme R₃ précédemment à l'exception de porter un substituant SOₓ-alcoyle ou alcoyloxy, ou bien R' et R'' peuvent le cas échéant être joints pour former un cycle à 5 ou 6 chaînons pouvant porter un autre hétéroatome tel que O, S(O)ₓ ou N éventuellement substitué par alcoyle, aryle ou arylméthyle, R° est H, acyloxyméthyle éventuellement substitué sur le méthyle comme N ci-dessus ou arylméthyle et R°° est aryle, hétéroaryle, cycloalcoyle, polyfluoroalcoyle ou alcoyle éventuellement substitué; ou bien
R₆ est H.

A titre d'exemple, des dérivés aromatiques substitués peuvent être notamment
1) des produits de formule générale : 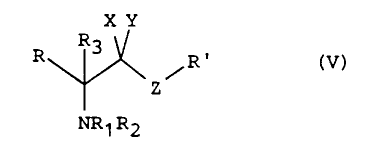 dans laquelle R₁ et R₂ sont indépendemment H, alcoyle pouvant être substitué (par OH, CN, CORa, COORa, CONRaRb, ou NRaRb), CORa, COORa ou CONRaRb dans laquelle Ra et Rb peuvent être H, alcoyle, phényle ou phénylalcoyle (1 à 4 carbones) (éventuellement substitués sur le cycle par alcoyle, alcoyloxy, halogène ou trifluorométhyle),
   R₃ est H ou alcoyle,
   X et Y sont H ou forment ensemble =O,
   Z est O, S ou NR'' pour lequel R'' est H ou alcoyle,
   R' est un radical -CHR°R°° pour lequel R° est -(CH₂)ₙ-phényle pouvant être substitué sur le cycle par 1 ou plusieurs alcoyle, alcényle ou alcynyle (2 à 6 carbones), halogène, CN, NO₂, CF₃, triméthylsilyle, ORa, SRa, SORa, SO₂Ra, NRaRb, NRaCORb, NRaCOORb, COORa ou CONRaRb dans lesquels Ra et Rb sont définis comme ci-dessus à l'exception d'être phényle substitué,
   a) ou bien R₁ et R₂ peuvent aussi être CONRaCOORb ou SO₂Ra,
      R°° est H, alcoyle, phényle éventuellement substitué comme défini pour R°, et n dans R° égale 0, et
      R est Q-CH₂- pour lequel Q est phényle, naphtyle, benzothiophényle, benzofuranyle, benzyle ou indazolyle éventuellement substitués;
   b) ou bien R₁ et R₂ peuvent aussi être CO-alcoyl-NRaRb, CONRaCOORb ou SO₂Ra,
      R₃ est défini comme ci-dessus pour la formule générale (V) ou représente alcényle,
      X et Y sont définis comme pour la formule générale (V) ou Y est OH, alcoyloxy, ou X et Y forment ensemble =NOR'',
      Z est un radical -CH₂- ou -CH=,
      R°° est H ou forme avec Z une double liaison et n dans R° égale 0, et R est Q-CH₂- pour lequel Q est phényle substitué par 1 ou plusieurs halogènes, ou naphtyle, indolyle, benzothiophényle, benzofuranyle benzyle ou fluorényle éventuellement substitués;
   c) ou bien R₁ et R₂ peuvent aussi être alcoyle substitué par CO-alcoyl-NRaRb ou CON(Ra)alcoylCONRaRb, phényle (éventuellement substitué par alcoyle, alcoyloxy, halogène ou trifluorométhyle), alcényle (2 à 6 carbones), halogénoacyle, CO-alcoyl-NRaRb, CON(Ra)alcoylCONRaRb, ou R₁ et R₂ forment ensemble un radical alcoylène à 4 ou 5 carbones dont un radical méthylène peut être remplacé par O ou NRx pour lequel Rx est H ou alcoyle,
      R°° est H, alcoyle, phényle éventuellement substitué comme défini pour R°, et n dans R° est 0 à 2, et
      R est un groupe : 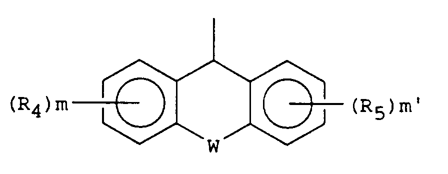 pour lequel W est une liaison, O, S, -(CH₂)₂-, -CH=CH-, ou NR'' et R4 et R₅ sont alcoyle, alcényle ou alcynyle (2 à 6 carbones), halogène, CN, NO₂, CF₃, triméthylsilyle, ORa, SRra, SORa, SO₂Ra, NRaRb, NRaCORb, NRaCOORb, COORa ou CONRaRb dans lesquels Ra et Rb sont définis comme ci-dessus à l'exception d'être phényle substitué et m et m' allant de 0 à 4;
   d) ou bien R₁ et R₂ peuvent aussi être alcoyle substitué par CO-alcoyl-NRaRb, CON(R'a)alcoylORa ou CON(R'a)alcoylCONRaRb, phényle (éventuellement substitué par alcoyle, alcoyloxy, halogène ou trifluorométhyle), alcényle (2 à 6 carbones), alcynyle (2 à 6 carbones), SO₂Ra, halogénoacyle, CO-alcoyl-NRaRb ou CON(Ra)alcoylCONRaRb, ou R₁ et R₂ forment ensemble un radical alcoylène à 4 ou 5 carbones dont un radical méthylène peut être remplacé par O ou NRx pour lequel Rx est H ou alcoyle et Ra et Rb sont définis comme précédemment pour la formule générale (V) ou forment ensemble une chaîne telle que définie pour R₁ et R₂ et R'a est défini comme Ra pour la formule générale (V),
      R₃ est défini comme ci-dessus pour la formule générale (V) ou représente alcényle,
      R°° est H et n dans R° est 0 à 2, et
      R est -CR₆R₇R₈ ou -CH₂-CR₆R₇R₈ pour lesquels R₆ est H ou OH et R₇ et R₈ sont phényle ou benzyle substitués, ou cycloalcoyle ou cycloalcoylméthyle (5 à 7 carbones);
   e) ou bien R₁ et R₂ peuvent aussi être définis comme ci-dessus en c) à l'exception de représenter simultanément H,
      X et Y sont définis comme pour la formule générale (V) ou peuvent être aussi alcoyle ou alcényle (2 à 6 carbones),
      Z est défini comme pour la formule générale (V) à l'exception d'être NR'',
      R°° et n dans R° sont définis comme ci-dessus en c) et
      R est phényle, naphtyle ou hétéroaryle éventuellement substitués; ou bien
2) des produits de formule générale : 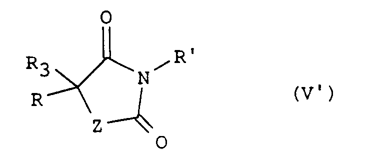 dans laquelle Z est O, S ou NR'' ou CR₄R₅ pour lesquels R'', R₄ et R₅ sont H, alcoyle ou, phényle ou phénylalcoyle (dont la partie alcoyle contient 1 à 4 carbones) éventuellement substitués, ou bien R'' est -COR₆, -COOR₆ ou -CONR₄R₅ et R₆ est alcoyle ou, phényle ou phénylalcoyle (dont la partie alcoyle contient 1 à 4 carbones) éventuellement substitués,
   R' est défini comme pour la formule (V),
   R₃ et R sont définis comme pour la formule générale (V) en b), et
   R°° est H et n dans R° est 0 à 3;
   étant entendu que, sauf mention spéciale, les portions alcoyle contiennent 1 à 6 carbones.

A titre d'exemple des antagonistes des récepteurs NK1 de la classe des dérivés dialcoylènepipéridino peuvent être des produits de formule générale : 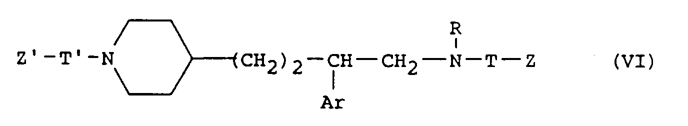 dans laquelle :
Ar est phényle éventuellement mono ou poly substitué (par halogène, alcoyle ou alcoyloxy (1 à 3 atomes de carbone), OH ou trifluorométhyle) ou thiényle, pyridyle ou naphtyle pouvant être substitués par halogène, ou benzothiényle ou indolyle, et
R est un atome d'hydrogène ou un radical alcoyle (1 à 6 atomes de carbone), ou aminoalcoyle de structure -(CH₂)ₙ-NH₂ pour lequel n égale 2 à 6,
T est un groupe -CO-, -CO-O-, -CO-NH- ou -CS-NH- , et
T' est une liaison sauf si Z' est un atome d'hydrogène, ou représente un radical -CH₂-, -CO-, -CH₂-O- ou -CO-O-,
Z et Z' identiques ou différents sont H, alcoyle droit ou ramifié (1 à 6 atomes de carbone), phénylalcoyle dont la partie alcoyle contient 1 à 3 atomes de carbone, éventuellement mono ou poly substitué sur le phényle par halogène, OH, alcoyle ou alcoyloxy (1 à 4 atomes de carbone), pyridylalcoyle ou naphtylalcoyle ou pyridylthioalcoyle ou (méthyl-1)imidazolyl-2 thioalcoyle dont la partie alcoyle contient 1 à 3 atomes de carbone, styryle, oxo-1 phénylindan-3 yle-2, ou un groupe aromatique ou hétéroaromatique non substitué, mono ou polysubstitué ou α-hydroxyhenzyle ou α-alcoylbenzyle dont la partie alcoyle contient 1 à 3 atomes de carbone;

Des produits de la classe des sels quaternaires de pipéridines substituées peuvent être notamment des produits de formule générale : 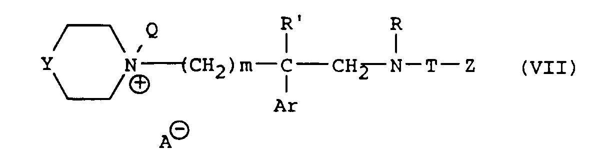 dans laquelle :
Y est Y'-CXX'-CX'' ou Y'-(CH₂)ₓ-CX° pour lesquels Y' est phényle pouvant être substitué (par 1 ou plusieurs H, halogène, OH, alcoyloxy, alcoyle ou CF₃), cycloalcoyle, pyridyle ou thiényle, X est H si X' forme une liaison avec X'', ou bien X et X' forment oxo et X'' est H, x est 0 et X° est H ou bien x est 0 ou 1 et X° est OH, alcoyloxy, acyloxy, carboxy, carbalcoyloxy, CN, -NH-CO-alcoyle, mercapto ou alcoylthio, ou bien X° forme une liaison avec l'atome de carbone de la pipéridine,
Q est alcoyle ou benzyle, R' est H et R est H ou alcoyle, ou bien Y est Y'-CX° pour lequel Y' est défini comme précédemment, X° forme avec Q un radical éthylényle et, R' et R forment ensemble avec les atomes auquels ils sont rattachés, un radical pipéridino,
A⁻ est un anion Cl⁻, Br⁻, I⁻, acétate, méthanesulfonate, p.toluènesulfonate,
Ar est défini comme pour la formule générale (VI) à l'exception de pyridyle ou naphtyle substitué, ou représente N-alcoyl indolyle,
T est C=O, CO-O, -(C=O)-NH- ou -(C=S)-NH-, et
Z est défini comme pour la formule générale (VI) ou représente α-hydroxyalcoylbenzyle ou phényle substitué par CF₃, naphtyle substitué par des substituants tels qu'énumérés pour phényle ou un groupe aromatique ou hétéroaromatique mono, di ou tricyclique éventuellement substitué et
m est 2 ou 3;
et parmi ces sels quaternaires notamment le produit de formule :

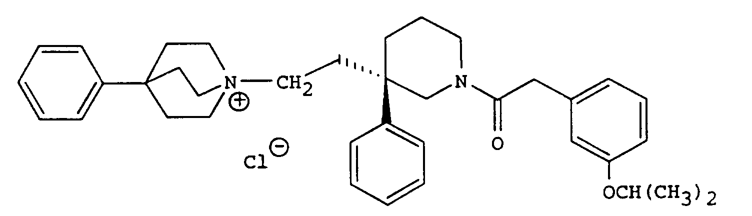

A titre non limitatif, des antagonistes des récepteurs NK2 peuvent être notamment des dérivés de la classe des arylalkylamines, la classe des polypeptides α-substitués ou de la classe des dérivés de la pipéridine ...

A titre d'exemple des antagonistes des récepteurs NK2 de la classe des arylalkylamines peuvent être des produits de formule générale : 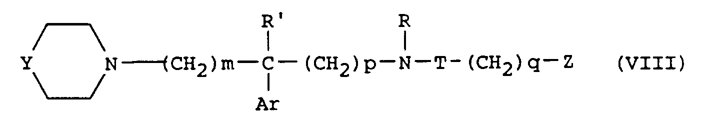 dans laquelle :
1) Y est un groupe >N-CXX'-Ar', >CH-CXX'-Ar' ou >C=CX-Ar' pour lequel X est H et X' est H ou OH ou X et X' forment ensemble un radical oxo, dialcoylaminoalcoyloxyimino pour lequel les parties alcoyle contiennent 1 à 4 atomes de carbone et la partie alcoyloxy contient 2 ou 3 atomes de carbone,
   Ar et Ar' sont indépendemment thiényle, phényle éventuellement mono ou poly substitué (par halogène, alcoyle ou alcoyloxy (1 à 3 atomes de carbone), trifluorométhyle, hydroxy ou méthylènedioxy) ou imidazolyle, ou bien Ar peut être benzothiényle ou naphtyle éventuellement substitués par halogène, biphényle, ou indolyle pouvant porter un groupe benzyle sur l'atome d'azote,
   R' est un atome d'hydrogène, un radical alcoyle (1 à 4 atomes de carbone), un radical alcoyle (2 ou 3 atomes de carbone) substitué (par pipéridino, benzyl-4 pipéridino ou dialcoylamino dont les parties alcoyle contiennent 1 à 4 atomes de carbone,
   R et T sont définis comme pour la formule générale (VI) et
   Z est H, alcoyle droit ou ramifié (1 à 6 atomes de carbone), phénylalcoyle dont la partie alcoyle contient 1 à 3 atomes de carbone, éventuellement mono ou poly substitué sur le phényle par halogène, OH, alcoyle ou alcoyloxy (1 à 4 atomes de carbone), pyridylalcoyle ou naphtylalcoyle ou pyridylthioalcoyle ou (méthyl-1)imidazolyl-2 thioalcoyle dont la partie alcoyle contient 1 à 3 atomes de carbone, styryle, oxo-1 phénylindan-3 yle-2, ou un groupe aromatique ou hétéroaromatique non substitué, mono ou polysubstitué, et
   m est un nombre entier de 1 à 3, p égale 1 et q égale 0, ou bien
2) Y est un groupe >N-Ar' pour lequel Ar' est un radical phényle pouvant être substitué 1 ou plusieurs fois (par halogène, OH, alcoyloxy ou alcoyle (1 à 4 atomes de carbone) ou trifluorométhyle), pyrimidinyle ou pyridyle,
   un groupe >N-cycloalcoyle (3 à 7 atomes de carbone), ou bien
   un groupe >CX-(CH₂)ₓ-Ar' pour lequel Ar' est défini comme ci-dessus à l'exception de représenter pyrimidinyle, ou représente thiényle, X est OH, alcoyloxy (1 à 4 atomes de carbone), hydroxyalcoyle ou acyloxy dont la partie alcoyle contient 1 à 3 atomes de carbone, phénacyloxy, carboxy, carbalcoyloxy (1 à 4 atomes de carbone), cyano, aminoalcoylène (1 à 3 atomes de carbone), amino, alcoylamino ou dialcoylamino dont les parties alcoyle contiennent 1 à 4 atomes de carbone, acylamino (2 à 7 atomes de carbone), acylaminoalcoyle dont les parties alcoyle contiennent 1 à 3 atomes de carbone, acyle, -SH, alcoylthio dont la partie alcoyle contient 1 à 4 atomes de carbone et x est 0 ou 1, ou bien
   un groupe =C-(CH₂)ₓ-Ar' dans lequel Ar' est défini comme ci-dessus,
   T est défini comme en 1), et Z est défini comme en 1) à l'exception de représenter (méthyl-1)imidazolyl-2 thioalcoyle ou oxo-1 phénylindan-3 yle-2, ou représente phénylalcoyle substitué par trifluorométhyle ou naphtylalcoyle dont la partie alcoyle contient 1 à 3 atomes de carbone et éventuellement substitué sur le cycle naphtyle par un atome d'halogène ou par un radical trifluorométhyle, OH, alcoyle ou alcoyloxy (1 à 4 atomes de carbone),
   Ar est thiényle, phényle éventuellement mono ou poly substitué (par halogène, alcoyle ou alcoyloxy (1 à 4 atomes de carbone), trifluorométhyle) ou benzothiényle, naphtyle ou indolyle pouvant porter un groupe alcoyle (1 à 3 atomes de carbone) sur l'atome d'azote,
   R' est un atome d'hydrogène,
   R est un atome d'hydrogène ou un radical alcoyle (1 à 6 atomes de carbone), et
   m est un nombre entier égal à 2 ou 3, p égale 1 et q égale 0, ou bien
3) Y est un groupe >N-Ar' ou >N-CH₂-Ar' pour lequel Ar' est défini comme ci-dessus en 2), ou bien
   un groupe >CX-(CH₂)ₓ-Ar' tel que défini ci-dessus, X étant OH, alcoyloxy, acyloxy ou carbalcoyloxy (1 à 4 atomes de carbone), carboxy, cyano, amino éventuellement mono ou di substitué (par alcoyle, hydroxyalcoyle ou acyle (1 à 4 atomes de carbone)), pyrrolidino, pipéridino, ou morpholino, -SH ou alcoylthio dont la partie alcoyle contient 1 à 4 atomes de carbone et x est 0 ou 1,ou bien
   un groupe =C-(CH₂)ₓ-Ar' dans lequel Ar' est défini comme ci-dessus,
   Ar est défini comme ci-dessus en 2) à l'exception de représenter un radical indolyle substitué,
   R et R' forment ensemble une chaîne 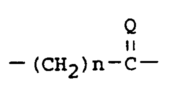 dans laquelle Q est un atome d'oxygène ou 2 atomes d'hydrogène,
   T est -CO- ou -CH₂- et
   Z est phényle ou naphtyle éventuellement mono ou polysubstitués par halogène, trifluorométhyle, OH ou alcoyle (1 à 4 carbones), ou par alcoyloxy (1 à 4 carbones) lorsque Z est phényle, pyridyle, thiényle, indolyle, quinoléyle, benzothiényle ou imidazolyle, ou un groupe aromatique ou hétéroaromatique non substitué, mono ou polysubstitué, ou lorsque T est CO, -(CH₂)q-Z peut être benzyle dont le radical méthyle est substitué par OH, alcoyle ou alcoyloxy (1 à 4 carbones), ou substitué sur le cycle par halogène, trifluorométhyle, OH, alcoyle ou alcoyloxy (1 à 4 carbones), et
   m est un nombre entier égal à 2 ou 3, p égale 1 ou 2, n égale 0 à 3 et q égale 0 à 3, étant entendu que si p=2 : n=1 et Q représente 2H; ou bien
4) Y est un groupe Ar'-X- pour lequel Ar' représente thiényle, phényle éventuellement mono ou poly substitué (par halogène, alcoyle ou alcoyloxy (1 à 3 carbones), trifluorométhyle, hydroxy ou méthylènedioxy) pyridyle ou imidazolyle éventuellement substitué par un radical alcoyle, et X est un atome d'oxygène ou de soufre ou un radical sulfonyle, sulfinyle, -NH-, >N-CO-Alk, >N-Alk, >N-Alk-NX₁X₂ peur lesquels Alk est alcoyle ou alcoylène (1 à 3 carbones) et X₁ et X₂ sont H, alcoyle (1 à 3 carbones) ou forment avec l'atome d'azote un cycle pipéridine, pyrrolidine ou morpholine,
   R' est un atome d'hydrogène, un radical alcoyle (1 à 4 carbones) ou un radical aminoalcoyle dont la partie alcoyle en chaîne droite contient 2 ou 3 atomes de carbone et le groupe amino peut être dialcoylamino dont les parties alcoyle contiennent 1 à 4 atomes de carbone, pipéridino, ou benzyl-4 pipéridino,
   Ar, R et T sont définis comme en 1),
   Z est défini comme précédemment 1) ou représente α-hydroxybenzyle ou α-alcoylbenzyle dont la partie alcoyle contient 1 à 3 atomes de carbone, et
   m, p et q sont définis comme précédemment en 2), ou bien
5) Y est un groupe >CX-(CH₂)ₓ-Ar' pour lequel Ar' est thiényle, phényle éventuellement mono ou poly substitué (par halogène, alcoyle ou alcoyloxy (1 à 4 carbones), trifluorométhyle, hydroxy) ou pyridyle, x est 0 ou 1, et X est -NH-CO-alcoyle don la partie alcoyle contient 1 à 6 carbones,
   m est 2 ou 3, p est 1 et q égale 0
   Ar, T et Z sont définis comme en 2),
   R' est H et R est H ou alcoyle;
   et parmi ces produits, le produit de l'exemple 2 mentionné ci-après, qui est un antagoniste spécifique des récepteurs NK2 est décrit plus particulièrement par X. Emonds-Alt et Coll., Life Science, 50, PL 100 à PL 106 (1992).

A titre d'exemple des antagonistes des récepteurs NK2 de la classe des polypeptides α-substitués peuvent être des produits de formule générale : 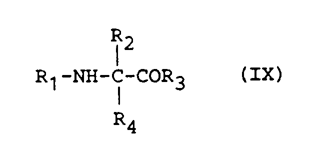 dans laquelle
R₁ est un atome d'hydrogène ou un groupe N-terminal constitué de 0 à 4 amino-acides,
R₂ est une chaine latérale d'amino-acide excepté la glycine,
R₃ est un groupe C-terminal constitué de 0 à 4 amino-acides, un radical OH ou OR dans laquelle R est un radical alcoyle droit ou ramifié ou cycloalcoyle contenant 1 à 6 atomes de carbone,
R₄ est une chaine latérale d'amino-acide excepté la glycine, ou un radical -CH=CH₂, -CH≡CH, -CH₂-CH=CH₂, -CH₂-CH≡CH, -CH₂-Ar, -CH₂-OR, -CH₂-OAr, -(CH₂)ₙCO₂R ou -CH₂-NR₅R₆, n étant un entier de 0 à 3, R étant H ou un radical alcoyle inférieur et Ar étant un carbocycle ou un hétérocycle aromatique ou hydroaromatique mono ou polycyclique non substitué ou substitué,
étant entendu que R₁ et R₃ ne peuvent pas comprendre plus de 4 résidus d'amino-acides en tout.

A titre d'exemple des antagonistes des récepteurs NK2 de la classe des dérivés de pipéridine peuvent être des produits de formule générale : 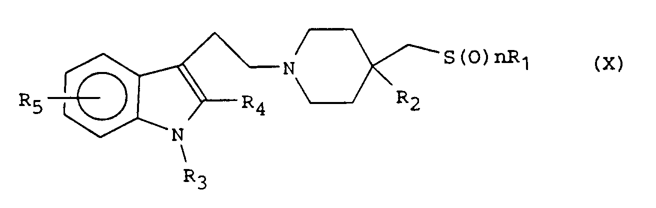 dans laquelle R₁ est phényle éventuellement substitué par 1 ou 2 alcoyle, alcoyloxy, CF₃, ou halogène,
R₂ est H, OH ou alcoyle,
R₃ est H ou alcoyle,
R₄ est H, alcoyle ou alcoyloxy, et
R₅ est H, alcoyle, CF₃, CN ou halogène et n est 0 à 2, les radicaux alcoyle ayant 1 à 4 carbones;
et parmi ces produits plus spécialement le 1-[2-(5-fluoro-1H-indol-3-yl) éthyl]-4-[(phénylsulfinyl)méthyl]-4-pipéridinol.

Les produits de formules générales (I) à (X) peuvent être préparés selon les méthodes décrites dans les demandes de brevet EP 429 366, EP 514 273, EP 514 275, WO 90/05525, WO 90/05729, WO 91/18899, WO 91/09844, WO 92/01688, WO 92/06079, WO 92/15585, WO 92/12151, WO 92/20661, WO 92/20676, WO 92/21677, WO 93/00330, WO 93/00331, WO 93/01159, WO 93/01169, WO 93/01165, WO 93/01170, WO 93/06099, WO 93/09116, WO 93/10073, WO 93/18023, WO 93/19064, WO 93/21155, WO 93/21181, WO 93/23380, EP 499 313, EP 394 989, EP 443 132, EP 482 539, EP 517 589, EP 520 555, EP 522 808, EP 528 495, EP 532 456, EP 533 280, EP 536 817, EP 545 478, EP 559 538, WO 91/18878, WO 92/17449, EP 428 434, EP 474 561, EP 512 901, EP 512 902, EP 515 240, FR 2 678 267, WO 92/19254, WO 93/14084, ou WO 93/21155 ou par analogie avec ces méthodes.

Le dérivé de thiapyranopyrrole de formule générale : 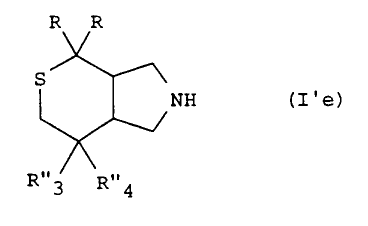 dans laquelle les radicaux R, R''₃ et R''₄ sont définis comme précédemment, utilisé comme matière de départ pour la préparation des produits dont le bicycle A-B répond à la formule générale (Ie) peut être préparé selon les méthodes suivantes :
- lorsque R''₄ est un atome de fluor le produit de formule générale (I'e) peut être préparé par fluoration d'un dérivé de thiapyranopyrrole de formule générale : 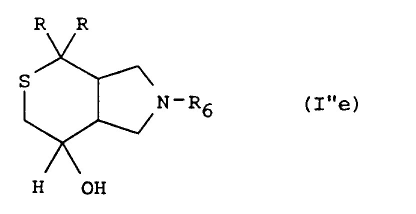 dans laquelle R est défini comme précédemment et R₆ est un radical protecteur, puis élimination du radical R₆^{.}

Le radical protecteur R₆ peut être tout groupement protecteur d' amino qui soit compatible avec la réaction et dont la mise en place et l'élimination n'altère pas le reste de la molécule. A titre d'exemple peuvent être cités les groupements alcoyloxycarbonyle, benzyloxycarbonyle, benzyle éventuellement substitués, formyle, chloracétyle, trichloracétyle, trifluoracétyle, vinyloxycarbonyle, phénoxycarbonyle, chloro-1 éthoxycarbonyle ou chlorocarbonyle.

La réaction s'effectue avantageusement au moyen d'un agent de fluoration comme un fluorure de soufre [trifluorure de morpholino soufre, tétrafluorure de soufre (J. Org. Chem., 40, 3808 (1975)), trifluorure de diéthylamino soufre (Tetrahedron, 44, 2875 (1988)), trifluorure de phényl soufre (J. Am. Chem. Soc., 84, 3058 (1962)], comme le tétrafluorure de sélenium (J. Am. Chem. Soc., 96,925 (1974) ou comme le tétrafluorophénylphosphorane (Tet. Let., 907 (1973), en opèrant dans un solvant organique tel qu'un solvant chloré (dichlorométhane, dichloréthane par exemple) à une température comprise entre -30 et 30°C. L'élimination subséquente du radical protecteur R₆ s'effectue selon les méthodes habituelles. Notamment selon les méthodes décrites par T.W. Greene, Protective Groups in Organic Synthesis, A. Wiley - Interscience Publication (1981), ou par Mc Omie, Protective Groups in Organic Chemistry, Plenum Press (1973).
- lorsque R''₃ est un radical phényle éventuellement substitué en position -2 par un radical alcoyle ou alcoyloxy et R''₄ est un radical hydroxy le produit de formule générale (I'e) peut être obtenu par action d'un composé organométallique de formule générale :

   R''₃-M (I'''e)

   dans laquelle R''₃ est défini comme précédemment, et M représente le lithium, le cérium ou un radical MgX pour lequel X est un atome d'halogène, sur le dérivé correspondant de thiapyranopyrrolone de formule générale : 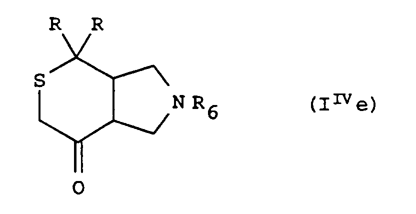 dans laquelle R et R₆ sont définis comme précédemment, puis éventuellement élimination du radical protecteur R₆.

La réaction s'effectue en milieu anhydre, dans les conditions habituelles de réaction des composés organométalliques sur une cétone, qui n'affectent pas le reste de la molécule. Notamment dans un éther (par exemple le tétrahydrofuranne ou l' éther éthylique) éventuellement en présence de chlorure de cérium anhydre à une température comprise entre -78 et 30°C.

Les dérivés de thiapyranopyrrole de formule générale (I'e) ou (I''e) pour lequel R''₃ est un atome d'hydrogène et R''₄ est un radical hydroxy, peuvent être obtenus par réduction respectivement du dérivé de formule générale (I^{IV}e) ou du dérivé correspondant de thiapyranopyrrolone de formule générale (I'e) pour lequel R₃ et R₄ forment ensemble un radical oxo. La réduction s'effectue avantageusement au moyen d'un borohydrure alcalin (borohydrure de sodium, tris.butylborohydrure de lithium) dans un solvant tel qu'un alcool (méthanol, éthanol par exemple) ou un éther (tétrahydrofuranne) en milieu basique, à une température comprise entre -20 et 50°C.

Le dérivé de la thiapyranopyrrolone de formule générale (Ie) pour laquelle R''₃ et R''₄ forment ensemble un radical oxo, ou le dérivé de la thiapyranopyrrolone de formule générale (I^{IV}e) peuvent être préparés à partir de la thiapyranone de formule générale : 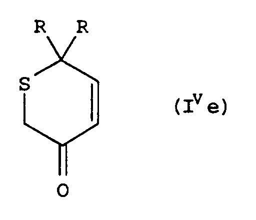 dans laquelle R est défini comme précédemment, puis mise en place du radical protecteur R₆ si l'on veut obtenir un produit de formule générale (I^{IV}e).

La potentialisation des associations d'antagonistes des récepteurs NK1 et d'antagonistes des récepteurs NK2 a été mise en évidence de la manière suivante :

La capsaïcine, administrée à faible dose, provoque au niveau des fibres afférentes primaires de différents tissus périphériques la libération de divers neuropeptides, notamment la substance P et la neurokinine A.

Les antagonistes de la substance P sont connus pour bloquer les effets d'un agoniste des récepteurs NK1 et les antagonistes de la neurokinine A sont connus pour bloquer les effets d'un agoniste des récepteurs NK2. Cependant lorsque ces agonistes sont remplacés par la capsaïcine l'action habituellement observée avec les agonistes devient extrèmement faible ou disparaît complètement.

Il a été montré, dans un test identique, que l'administration d'une association de produits antagonistes de la substance P et antagonistes de la neurokinine A bloque les effets de la capsaïcine, ou en d'autres termes fait réapparaître l'action habituellement observée avec les agonistes NK1 et NK2.

### METHODES :

### I/ INHIBITION DE LA CONTRACTION DE LA VESSIE DE RAT, IN VIVO, DUE A UNE ADMINISTRATION TOPIQUE DE CAPSAICINE

### PRINCIPE

L'application locale de capsaïcine sur la vessie de rat, in vivo, déclenche une contraction dose-dépendante due principalement à une libération de substance P et de neurokinine A agissant respectivement sur les récepteurs NK1 et NK2. Un prétraitement par un antagoniste NK1 ou NK2, ou leur association, devrait donc inhiber la contraction submaximale provoquée par la capsaïcine.

### TECHNIQUE

Les animaux utilisés sont des rats femelles Sprague-Dawley pesant de 210 à 260 g qui sont anesthésiés par du carbamate d'éthyle et dont la température rectale est maintenue à 380°C. On effectue un prétraitement par la chlorisondamine (Ecolid®) 0,5mg/kg i.v. pour supprimer toute contraction vésicale spontanée ou réflexe. La pression intravésicale est mesurée en continu grâce à un cathéter introduit par voie uréthrale et relié à un capteur de pression à jauge de contrainte lui-même connecté à un amplificateur et à un enregistreur graphique. Cette pression est ajustée à une valeur de 3mm de mercure en début d'expérience par remplissage avec du soluté de NaCl à 0,9 %. On effectue une laparotomie basse pour accéder à la vessie.

Chaque antagoniste (NK1 ou NK2) ou leur association est injectée dans une veine jugulaire 40 minutes après l'administration de chlorisondamine.

Cinq minutes après le traitement par le ou les antagonistes, une solution de capsaïcine (250 ng dans 10 ml) est appliquée sur le dôme de la vessie pendant 3 minutes, grâce à son dépôt sur un petit morceau de papier filtre préalablement placé sur cette région. Le % d'inhibition, par le ou les antagonistes, de la contraction due à la capsaïcine est calculé en comparant l'élévation de la pression intravésicale observée chez les lots traités par rapport au lot témoin capsaïcine.

### RESULTATS :

Les produits étudiés sont désignés comme défini ci-après:
- Antagoniste des récepteurs NK1 :
   Produit 1 : [imino-1 (méthoxy-2 phényl)-2 éthyl]-2 diphényl-7,7 perhydroisoindolone-4-(3aR,7aR) : 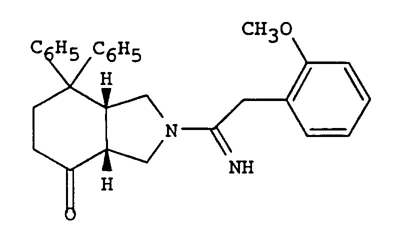
- Antagoniste des récepteurs NK2 :
   - Produit 2 :: N-méthyl [(phényl-4 acétamido-4 pipéridinyl)-4 (dichloro-3,4 phényl)-2 butyl] benzamide :

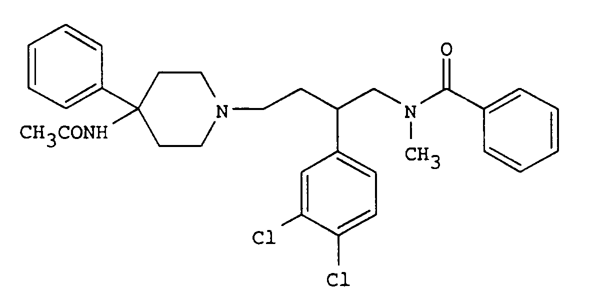

**Tableau I**

| Traitement | Δ pression intravésicale (mm de Hg) | Inhibition par rapport au lot témoin |
|---|---|---|
| Témoins capsaïcine 250 ng par voie topique | 5,5 ±0,9 | |
| Produit 1 | 5,3+1,1 (non significatif) | 4 % |
| Produit 2 | 5,0+0,6 (non significatif) | 9 % |
| Produit 1 + Produit 2 | 1,4+0,2 (P<0,001) | 74 % |

Le produit 1 et le produit 2 administrés seuls à la dose de 0,5 mg/kg par voie intra-veineuse n'ont pas inhibé la contraction provoquée par la capsaïcine. Par contre leur association a nettement diminué (74 %) l'action de la capsaïcine et ce de façon tout à fait significative (P<0,001).

### II/ INHIBITION DU BRONCHOSPASME PROVOQUE PAR LA CAPSAICINE ADMINISTREE PAR VOIE INTRA-VEINEUSE CHEZ LE COBAYE

### PRINCIPE

L'injection i.v. de capsaïcine chez le cobaye déclenche un bronchospasme dû principalement à une libération de substance P et de neurokinine A agissant respectivement sur les récepteurs NK1 et NK2. Un prétraitement par un antagoniste NK1 ou NK2, ou leur association, devrait donc inhiber ce bronchospasme.

### TECHNIQUE

Les animaux utilisés sont des cobayes femelles Hartley pesant de 330 à 480 g anesthésiés par du carbamate d'éthyle et dont la température rectale est maintenue à 370°C. Après mise en route d'une respiration artificielle (0,1 ml d'air/100 g de poids corporel, 50 insufflations/min), les animaux reçoivent un curarisant (bromure de pancuronium 0,8mg/kg i.v.) pour obtenir une meilleure ventilation, puis de la chlorisondamine (Ecolid®) 0,5 mg/kg i.v. pour bloquer les réflexes du système nerveux autonome. La pression d'insufflation est mesurée en continu dans une tubulure latérale placée sur la canule trachiale grâce à un capteur de pression à jauge de contrainte connecté à un amplificateur et à un enregistreur graphique.

La capsaïcine (2,5 mg/kg i.v. bolus) est injectée 25 minutes avant, puis 5 minutes après le traitement i.v. par l'antagoniste (NK1 ou NK2) ou leur association. Toutes ces injections sont effectuées dans une veine jugulaire.

Le % d'inhibition, par le ou les antagonistes, du bronchospasme dû à la capsaïcine est calculé en comparant l'augmentation de la pression maximale d'insufflation intrachiale observée à la deuxième injection de capsaïcine par rapport à la première.

### RESULTATS

Les produits étudiés sont désignés comme défini ci-après :
- Antagoniste des récepteurs NK1 :
   Produit 3 : diphényl-7,7 (méthoxy-2 phényl)-4 [(méthoxy-2 phényl)-2 propionyl-(S)]-2 perhydroisoindolol-4-(3aS,4S,7aS) 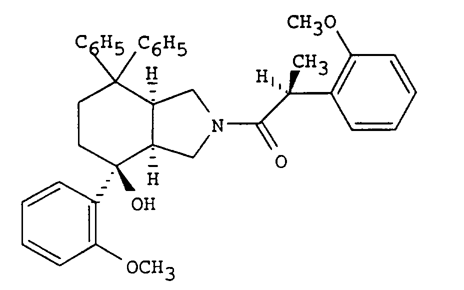
   Produit 4 : [(méthoxy-2 phényl) méthylamino]-3 phényl-2 pipéridine 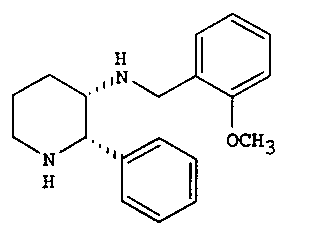
- Antagoniste des récepteurs NK2 :
   Produit 2 : N-méthyl [(phényl-4 acétamido-4 pipéridinyl)-4 (dichloro-3,4 phényl)-2 butyl] benzamide.

**Tableau II**

| Traitement | Δ pression d'insufflation dû à la capsaïcine (mm de Hg) | | Variation en % de la réponse à la capsaïcine Après/avant traitement |
|---|---|---|---|
| | Avant traitement | Après traitement | |
| Témoins capsaïcine 250 ng par voie i.v. | 26 ± 5 | 27 ± 4 (non significatif) | + 4 % |
| Produit 3 | 26 ± 3 | 25 ± 2 (non significatif) | - 4 % |
| Produit 2 | 27 ± 3 | 26 ± 5 (non significatif) | - 4 % |
| Produit 2 + 3 | 34 ±3 | 11 ± 2 (P < 0,001) | - 68 % |
| Produit 4 | 25 ± 5 | 28 ± 7 (non significatif) | +13 % |
| Produit 2 | 27 ± 3 | 26 ± 5 (non significatif) | - 4 % |
| Produits 2 + 4 | 23 ± 6 | 0,5 ± 0,2 (P < 0,001) | - 98 % |

Les produits 3 et 4 administrés seuls à la dose de 1 mg/kg par voie intra-veineuse n'ont pas inhibé le bronchospasme provoqué par la capsaïcine. Il en est de même pour le produit 2 (antagoniste NK2) à la même dose. Par contre l'association des produits 2 et 3 a diminué le bronchospasme de 68 % et l'association des produits 2 et 4 a diminué le bronchospasme de 98 %.

Ainsi comme l'indiquent les tableaux I et II, aucun des antagonistes administré seul n'est capable d'inhiber les effets de la capsaïcine. Au contraire l'association d'antagoniste des récepteurs NK1 et NK2 antagonise puissamment les effets spasmogènes de la capsaïcine sur la vessie et les bronches.

L'exemple suivant illustre la préparation de produits pouvant être utilisés dans l'association.

### Préparation du produit 1

A une suspension de 0,8 g de chlorhydrate de diphényl-7,7 (méthoxy-2 phényl)-4 perhydroisoindolol-4-(3aS,4S,7aS) dans 60 cm³ de dichlorométhane sec on ajoute 0,025 g d'hydroxy-1 benzotriazole, 0,38 g d'acide (méthoxy-2 phényl)-2 propionique-(S), 0,32 cm³ de diisopropyléthylamine, puis on refroidit cette solution à +5°C et on ajoute rapidement une suspension de 0,43 g de chlorhydrate de (diméthylamino-3 propyl)-1 éthyl-3 carbodiimide dans 10 cm³ de dichlorométhane sec. Le mélange réactionnel est agité 2 heures à +5°C, 2 heures à température ambiante, lavé par 20 cm³ d'eau, puis lavé par 20 cm³ d'une solution aqueuse saturée en chlorure de sodium (deux fois), séché sur sulfate de magnésium, et concentré à sec sous pression réduite (2,7 kPa). Le résidu est chromatographié sur colonne de gel de silice Merck (granulométrie 0,04-0,06 mm, diamètre 2,8 cm, hauteur 20 cm), en éluant sous une pression de 0,5 bar d'azote par un mélange de cyclohexane et d'acétate d'éthyle (80/20 en volumes) et en recueillant des fractions de 25 cm^{3.} Les fractions 9 à 15 sont réunies puis concentrées à sec sous pression réduite (2,7 kPa). Le résidu est cristallisé dans un mélange d'acétonitrile et d'oxyde de diisopropyle. On obtient 0,17 g de diphényl-7,7 (méthoxy-2 phényl)-4 [(méthoxy-2 phényl)-2 propionyl-(S)]-2 perhydroisoindolol-4-(3aS,4S,7aS) sous la forme de cristaux blancs fondant à 244°C.

Le chlorhydrate de diphényl-7,7 (méthoxy-2 phényl)-4 perhydroisoindolol-4-(3aS,4S,7aS) peut être préparé de la manière suivante :

A une solution de 7,63 g de diphényl-7,7 (méthoxy-2 phényl)-4 tert-butoxycarbonyl-2 perhydroisoindolol-4-(3aS,4S,7aS) dans 66 cm³ de dioxanne on ajoute à température ambiante une solution de 100 cm³ de dioxanne chlorhydrique 5,2 N. Le mélange réactionnel est agité 1 heure à cette température, puis concentré à sec sous pression réduite (2,7 kPa). Le résidu est lavé par de l'acétonitrile, essoré puis séché. On obtient 4,88 g de chlorhydrate de diphényl-7,7 (méthoxy-2 phényl)-4 perhydroisoindolol-4-(3aS,4S,7aS) sous la forme de cristaux blancs fondant à 271°C (bloc Maquenne).

Le diphényl-7,7 (méthoxy-2 phényl)-4 tert-butoxycarbonyl-2 perhydroisoindolol-4-(3aS,4S,7aS) peut être préparé de la manière suivante :

A une suspension de 20 g de diphényl-7,7 tert-butoxycarbonyl-2 perhydroisoindolone-4-(3aS,7aS) et de 31,6 g de chlorure de cérium anhydre dans 250 cm³ de tétrahydrofuranne sec, on ajoute goutte à goutte à température ambiante sous agitation une suspension de bromure de méthoxy-2 phénylmagnésium (préparée à partir de 75,3 g de bromo-2 anisole et de 9,8 g de magnésium) dans 100 cm³ de tétrahydrofuranne sec. Le mélange réactionnel est agité à température ambiante pendant 24 heures, traité par 400 cm³ d'une solution aqueuse saturée en chlorure d'ammonium, dilué par 200 cm³ d'acétate d'éthyle, lavé par 300 cm³ d'eau (deux fois), puis par 300 cm³ d'une solution aqueuse saturée en chlorure de sodium, séché sur sulfate de magnésium, filtré et concentré à sec sous pression réduite (2,7 kPa). Le résidu est chromatographié sur colonne de gel de silice Merck (granulométrie 0,04-0,06 mm, diamètre 5,8 cm, hauteur 26,5 cm), en éluant sous une pression de 0,5 bar d'azote par un mélange de cyclohexane et d'acétate d'éthyle (80/20 en volumes) et en recueillant des fractions de 100 cm³. Les fractions 9 à 29 sont réunies et concentrées à sec sous pression réduite (2,7 kPa). On obtient 17,82 g de diphényl-7,7 (méthoxy-2 phényl)-4 tert-butoxycarbonyl-2 perhydro-isoindolol-4-(3aS,4S,7aS) sous forme d'une meringue blanche.

Spectre RMN du proton (DMSO d₆) : 1,36 (s, 9H, -C(CH₃)₃); 1,54 (dmt, J=14, 1H, H équatorial du -CH₂- en 5); 2,3 (dmt, J=14, 1H, H équatorial du -CH₂- en 6); 2,34 (td, J=14 et 2,5, 1H, H axial du -CH₂- en 5); 3,07(td, J=14 et 2,5, H axial du -CH₂-en 6); 3,49(s, 3H, -OCH₃); 2,6 à 3,6(mt, autre -CH₂- et -CH); 6,85 à 7,7 (mt, 14H, aromatiques).

La diphényl-7,7 tert-butoxycarbonyl-2 perhydroisoindolone-4-(3aS,7aS) peut être obtenue de la manière suivante :

A une suspension de 80 g de chlorhydrate de diphényl-7,7 perhydroisoindolone-4-(3aS,7aS) dans 400 cm³ de dichlorométhane sec on ajoute à température ambiante sous agitation 34,3 cm³ de triéthylamine, 58,6 g de di-tert-butyl dicarbonate, puis 2,98 g de diméthylamino-4 pyridine. Le mélange réactionnel est agité à température ambiante pendant 24 heures, lavé par 100 cm³ d'une solution aqueuse d'acide citrique, puis par 100 cm³ d'une solution aqueuse d'hydrogénocarbonate de sodium, puis par 100 cm³ d'une solution aqueuse saturée en chlorure de sodium, séché sur sulfate de magnésium, et concentré à sec sous pression réduite (2,7 kPa). On obtient 106,5 g de diphényl7,7 tert-butoxycarbonyl-2 perhydroisoindolone-4-(3aS,7aS) sous la forme d'une meringue orange.

Spectre RMN du proton (DMSO d₆) : 1,4(s, 9H, -C(CH₃)₃); 2,11 (td, J=15 et 7,5, 1H, H axial du -CH₂-en 5); 2,3(dt, J=15 et 3,5, 1H, H équatorial du -CH₂- en 5); 2,75 à 2,9 (mt, 4H, -CH₂- en 6 et -CH₂- en 1); 3,26 (dd, J=7,5 et 7, 1H, -CH en 3a); 3,35 (dd, J=11 et 7, 1H, 1H du -CH₂- en 3); 3,97 (mt, 1H, -CH en 7a); 4,1 (d, J=11, 1H, l'autre H du -CH₂- en 3); 7,1 à 7,7 (mt, 10H, aromatiques).

Le chlorhyrate de diphényl-7,7 perhydroisoindolone-4-(3aS,7aS) peut être obtenu de la manière suivante :

A une suspension de 20 g de chlorhydrate de diphényl-7,7 perhydroisoindolone-4-(3aRS,7aRS) dans 250 cm³ d'acétate d'éthyle, on ajoute lentement, sous agitation, 50 cm³ de soude aqueuse 4N ; l'agitation est poursuivie jusqu'à disparition du produit de départ. La solution organique est lavée par 100 cm³ d'eau distillée, par 100 cm³ d'une solution saturée en chlorure de sodium, séchée sur sulfate de magnésium et filtrée. A la solution ainsi obtenue, on ajoute sous agitation une solution de 9,3 g d'acide D-(-) mandélique dans 50 cm³ d'acétate d'éthyle. Les cristaux formés sont filtrés essorés, lavés par 50 cm³ d'acétate d'éthyle (deux fois) et séchés. Les cristaux sont repris par une solution de 220 cm³ d'acétonitrile et de 60 cm³ d'eau distillée, le mélange est porté au reflux sous agitation pendant 15 minutes ; les cristaux formés sont filtrés, et à nouveau cristallisés dans un mélange de 100 cm³d'acétonitrile et de 35 cm³ d'eau distillée. On obtient 6,4 g de D-mandélate de diphényl-7,7 perhydroisoindolo-ne-4-(3aS,7aS).

A 6,4 g de D-mandélate de diphényl-7,7 perhydroisoindolone-4-(3aS,7aS) en solution dans 100 cm³ d'acétate d'éthyle on ajoute 50 cm³ de soude aqueuse 1N ; le mélange réactionnel est agité à température ambiante jusqu'à disparition du produit de départ ; la solution organique est lavée par 50 cm³ d'eau distillée, par 50 cm³ d'une solution aqueuse saturée en chlorure de sodium, séchée sur sulfate de magnésium et filtrée ; elle est acidifiée sous agitation par addition de 2 cm³ d'une solution 9N d'acide chlorhydrique dans l'éthanol ; les cristaux obtenus sont essorés, lavés par de l'acétate d'éthyle, puis par de l'oxyde d'isopropyle et séchés. On obtient 4,24 g de chlorhydrate de diphényl-7,7 perhydroisoindolone-4-(3aS,7aS) sous la forme de cristaux blancs fondant à 270°C avec décomposition.

L'acide (méthoxy-2 phényl)-2 propionique-(S) peut être préparé, par analogie avec les méthodes décrites par D.A Evans et coll., Tetrahedron, 44, 5525, (1988), selon le mode opératoire suivant:

A une solution refroidie à +5°C de 4,1 g de méthyl-4 phényl-5 [(méthoxy-2 phényl)-2-(S)-propionyl]-3 oxazolidinone-2-(4S,5S) dans 60 cm³ de tétrahydrofuranne et 30 cm³ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. [α]_{D}²⁰ = +84,6° (c=1 ; CHCl₃).

La méthyl-4 phényl-5 [(méthoxy-2 phényl)-2-(S)-propionyl]-3 oxazolidinone-2-(4S,5S)peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 cm³ de tétrahydrofuranne on ajoute 19,1 g d'hexaméthyl-1,1,1,3,3,3 disilazanate de sodium, on agite 45 minutes à cette température, puis on ajoute 7,72 cm³ d'iodure de méthyle. Le mélange réactionnel est ensuite agité 15 heures à température ambiante, puis dilué par de l'acétate d'éthyle, lavé par 50 cm³ d'eau, puis par 50 cm³ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl)-2-(S)-propionyl]-3 oxazolidinone-2- (4S,5S) sous la forme d'un solide blanc.

La méthyl-4 phényl-5 (méthoxy-2 phénylacétyl)-3 oxazolidinone-2-(4S,5S) peut être obtenue de la manière suivante :

A une suspension de 1,89 d'hydrure de sodium (dispersion à 80 % dans la vaseline) dans 200 cm³ de tétrahydrofuranne sec on ajoute à température ambiante 9,38 g d'acide méthoxy-2 phénylacétique. On refroidit cette suspension à -30°C, on ajoute 7,77 cm³ de chlorure de pivaloyle, puis on ajoute enfin une solution refroidie à -78°C obtenue en additionnant une solution de 35,27 cm³ de butyllithium 1,6 M dans l'hexane à une solution refroidie à -78°C de 10 g de méthyl-4 phényl-5 oxazolidinone-2-(4S,5S) dans 200 cm³ de tétrahydrofuranne sec. Le mélange réactionnel est agité 45 minutes à -30°C, puis après retour à température ambiante, on ajoute 200 cm³ d'une solution aqueuse saturée de chlorure d'ammonium, puis 500 cm³ d'acétate d'éthyle ; après décantation la phase organique est lavée deux fois par 100 cm³ d eau, puis deux fois par 100 cm³ d'une solution aqueuse saturée de chlorure de sodium ; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes) et en recueillant des fractions de 50 cm³. Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

Les associations selon l'invention sont particulièrement intéressantes dans les domaines où interviennent la substance P, la neurokinine A et les récepteurs NK1 et NK2. La présente invention concerne également les compositions pharmaceutiques comprenant l'association synergisante constituée d'au moins un antagoniste des récepteurs NK1 et d'au moins un antagoniste des récepteurs NK2 à l'état pur ou sous forme d'une association avec un ou plusieurs diluants et/ou adjuvants compatibles et pharmaceutiquement acceptables et/ou éventuellement en association avec tout autre produit pharmaceutiquement compatible physiologiquement actif.

Les compositions selon l'invention peuvent être utilisées par voie parentérale, orale, rectale ou topique.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont des suppositoires ou des capsules rectales, qui contiennent outre le produit actif des excipients tels que le beurre de cacao, des glycérides semi-synthétiques ou des polyéthylèneglycols.

Comme compositions solides pour administration orale peuvent être utilisés des gélules, des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

En thérapeutique humaine, les associations selon l'invention peuvent être particulièrement utiles dans le traitement des douleurs d'origine traumatique, post-chirurgicale, menstruelle, céphaliques, dans les traitements de l'anxiété, des psychoses, de la maladie de Parkinson, de la schizophrénie, de la maladie d'Alzheimer, dans les traitements myorelaxants, dans les traitements des manifestations spasmodiques, douloureuses et inflammatoires des voies digestives (colites ulcéreuses, syndrome du colon irritable, maladie de Crohn), des voies urinaires (cystites) et des voies respiratoires (bronchite chronique, asthme, rhinites) ou en gynécologie et dans les traitements des migraines. Les nouvelles associations sont également utiles dans le traitement de l'arthrite rhumatoïde et dans les troubles dus au dérèglement du système immunitaire, dans les traitements des inflammations en dermatologie tels que le psoriasis, l'herpès, les urticaires, les eczémas, les photodermatoses et dans les troubles inflammatoires oculaires ou dentaires.

Les associations selon l'invention peuvent également trouver une application dans les traitements des troubles cardiovasculaires tels que l'hypotension, ou dans le traitement des troubles liés à une mauvaise régulation de la croissance (nanisme, hypotrophies secondaires à des maladies chroniques de l'enfant, ostéoporose, développement de greffes).

Les doses dépendent de l'effet recherché et de la durée du traitement. Pour un adulte, elles sont généralement comprises entre 0,25 et 1500 mg de l'association par jour en prises échelonnées.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

### Exemple

On prépare, selon la technique habituelle, des comprimés de produit actif ayant la composition suivante :

| | |
|---|---|
| - diphényl-7,7 (méthoxy-2 phényl)-4 [(méthoxy-2 phényl)-2 propionyl-(S)]-2 perhydroisoindolol-4-(3aS,4S,7aS) | 12,5 mg |
| - N-méthyl [(phényl-4 acétamido-4 pipéridinyl)-4 (dichloro-3,4 phényl)-2 butyl] benzamide | 12,5 mg |
| - amidon | 83 mg |
| - silice | 30 mg |
| - stéarate de magnésium | 3 mg |

## Revendications

1. Une association synergisante caractérisée en ce qu'elle est constituée d'au moins un produit doué d'une activité antagoniste des récepteurs NK1 et d'au moins un produit de nature non peptidique, doué d'une activité antagoniste des récepteurs NK2.

2. Utilisation d'un produit doué d'une activité antagoniste des récepteurs NK1 pour la préparation d'une association synergisante selon la revendication 1.

3. Utilisation d'un produit de nature non peptidique, doué d'une activité antagoniste des récepteurs NK2 pour la préparation d'une association synergisante selon la revendication 1.

4. Composition pharmaceutique caractérisée en ce qu'elle est constituée d'une association selon la revendication 1 à l'état pur ou en présence de tout diluant ou adjuvant compatible et pharmaceutiquement acceptable.

5. Une association selon la revendication 1, caractérisée en ce qu'elle comprend au moins un antagoniste des récepteurs NK1 choisi parmi les dérivés de la classe des perhydroisoindoles, les dérivés de la classe des 3-amino-quinuclidines-2-substituées, les dérivés de la classe des amino-azabicycloalcanes, les dérivés de la classe des 3-amino-pipéridines-2-substituées, les dérivés de la classe des 1-aza-bicyclo[3.2.2]nonan-3-amine, les dérivés de la classe des sels de N-alcoyl-quinuclidinium, les dérivés de la classe des pseudopeptides, les dérivés de la classe des diacylpipérazines, des dérivés aromatiques substitués, des dérivés dialcoylènepipéridino ou des sels quaternaires de pipéridines substituées.

6. Une association selon la revendication 1, caractérisée en ce qu'elle comprend au moins un antagoniste des récepteurs NK2 choisi parmi les produits de la famille des arylalkylamines ou de la classe des dérivés de pipéridine.

## Claims

1. A synergistic combination, characterized in that it consists of at least one product endowed with an antagonist activity towards NK1 receptors and of at least one product of non-peptide nature endowed with an antagonist activity towards NK2 receptors.

2. Use of a product endowed with an antagonist activity towards NK1 receptors for the preparation of a synergistic combination according to claim 1.

3. Use of a product of non-peptide nature endowed with an antagonist activity towards NK2 receptors for the preparation of a synergistic combination according to claim 1.

4. Pharmaceutical composition, characterized in that it consists of a combination according to claim 1 in the pure state or in the presence of any compatible and pharmaceutically acceptable diluent or adjuvant.

5. A combination according to claim 1, characterized in that it comprises at least one antagonist of NK1 receptors chosen from derivatives of the perhydroisoindole class, derivatives of the 2-substituted-3-aminoquinuclidine class, derivatives of the aminoazabicycloalkane class, derivatives of the 2-substituted-3-amino-piperidine class, derivatives of the 1-azabicyclo[3.2.2]nonan-3-amine class, derivatives of the N-alkylquinuclidinium salt class, derivatives of the pseudopeptide class, derivatives of the diacylpiperazine class, substituted aromatic derivatives, dialkylenepiperidino derivatives or substituted piperidine quaternary salts.

6. A combination according to claim 1, characterized in that it comprises at least one antagonist of NK2 receptors chosen from the products of the arylalkylamine family or of the piperidine derivative class.

## Patentansprüche

1. Synergistische Assoziation, dadurch gekennzeichnet, daß sie aus mindestens einem Produkt mit einer antagonistischen Aktivität der Rezeptoren NK1 und aus mindestens einem Produkt nicht-peptidischer Beschaffenheit mit einer antagonistischen Aktivität der Rezeptoren NK2 besteht.

2. Verwendung eines Produktes mit einer antagonistischen Aktivität der Rezeptoren NK1 für die Herstellung einer synergistischen Assoziation nach Anspruch 1.

3. Verwendung eines Produktes nicht-peptidischer Beschaffenheit mit einer antagonistischen Aktivität der Rezeptoren NK2 für die Herstellung einer synergistischen Assoziation nach Anspruch 1.

4. Pharmazeutische Zusammensetzung, dadurch gekennzeichnet, daß sie aus einer Assoziation nach Anspruch 1 in reinem Zustand oder in Anwesenheit von jedem kompatiblen und pharmazeutisch akzeptablen Verdünnungsmittel oder Zusatzstoff besteht.

5. Assoziation nach Anspruch 1, dadurch gekennzeichnet, daß sie mindestens einen Antagonisten der Rezeptoren NK1 umfaßt, ausgewählt unter den Derivaten der Klasse der Perhydroisoindole, den Derivaten der Klasse der 2-substituierten 3-Amino-chinuclidine, den Derivaten der Klasse der Amino-azabicycloalkane, den Derivaten der Klasse der 2-substituierten 3-Amino-piperidine, den Derivaten der Klasse der 1-Aza-bicyclo[3.2.2]-nonan-3-amine, den Derivaten der Klasse der N-Alkyl-chinuclidinium-Salze, den Derivaten der Klasse der Pseudopeptide, den Derivaten der Klasse der Diacylpiperazine, den substituierten aromatischen Derivaten, den Dialkylenpiperidino-Derivaten oder den quaternären Salzen von substituierten Piperidinen.

6. Assoziation nach Anspruch 1, dadurch gekennzeichnet, daß sie mindestens einen Antagonisten der Rezeptoren NK2 umfaßt, ausgewählt unter den Produkten der Familie der Arylalkylamine oder der Klasse der Piperidin-Derivate.
